(19) Europäisches Patentamt
European Patent Office
Office européen des brevets

(11) Veröffentlichungsnummer: **0 336 354 B1**

(12) **EUROPÄISCHE PATENTSCHRIFT**

(45) Veröffentlichungstag der Patentschrift: **02.11.94**

(21) Anmeldenummer: **89105861.2**

(22) Anmeldetag: **04.04.89**

(51) Int. Cl.[5]: **C07D 239/42**, C07D 239/52, C07D 249/14, C07D 251/46, C07D 471/04, C07D 487/04, A01N 47/36

Die Akte enthält technische Angaben, die nach dem Eingang der Anmeldung eingereicht wurden und die nicht in dieser Patentschrift enthalten sind.

# (54) Heterocyclisch substituierte Alkyl- und Alkenylsulfonylharnstoffe, Verfahren zu ihrer Herstellung und ihre Verwendung als Herbizide oder Pflanzenwachstumsregulatoren.

(30) Priorität: **08.04.88 DE 3811777**

(43) Veröffentlichungstag der Anmeldung:
**11.10.89 Patentblatt 89/41**

(45) Bekanntmachung des Hinweises auf die Patenterteilung:
**02.11.94 Patentblatt 94/44**

(84) Benannte Vertragsstaaten:
**AT BE CH DE ES FR GB IT LI NL SE**

(56) Entgegenhaltungen:
**EP-A- 0 061 661**
**EP-A- 0 071 958**
**EP-A- 0 085 236**
**EP-A- 0 117 014**
**EP-A- 0 139 947**

(73) Patentinhaber: **Hoechst Schering AgrEvo GmbH**
**Gerichtstrasse 27**
**D-13342 Berlin (DE)**

(72) Erfinder: **Willms, Lothar, Dr.**
**Schulstrasse 3**
**D-5416 Hillscheid (DE)**
Erfinder: **Bauer, Klaus, Dr.**
**Doorner Strasse 53D**
**D-6450 Hanau (DE)**
Erfinder: **Bieringer, Hermann, Dr.**
**Eichenweg 26**
**D-6239 Eppstein/Taunus (DE)**
Erfinder: **Bürstell, Helmut, Dr.**
**Am Hohlacker 65**
**D-6000 Frankfurt am Main 50 (DE)**

**EP 0 336 354 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß heterocyclisch substituierte Alk(en)ylsulfonylharnstoffe herbizide und pflanzenwachstumsregulierende Eigenschaften aufweisen (s. EP-A 061,661; EP-A 071,958; EP-A 085,236; EP-A 0,139,947). Diese weisen jedoch zum Teil bei ihrer Anwendung Nachteile auf, wie beispielsweise eine hohe Persistenz oder unzureichende Selektivität in wichtigen Nutzkulturen.

Es wurden nun neue heterocyclische Sulfonylharnstoffe mit vorteilhaften herbiziden Eigenschaften gefunden.

Gegenstand der vorliegende Erfindung sind daher die Verbindungen der Formel (I) oder deren Stereoisomere

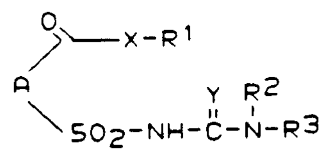

(I) ,

worin

A = einen Rest der Formel $>C(R^4)_2$ oder $>C=C(R^5)_2$,

$R^1$ = H, $(C_1$-$C_8)$Alkyl, $(C_2$-$C_8)$Alkenyl, $(C_2$-$C_8)$Alkinyl, wobei diese aliphatischen Reste ein- oder mehrfach durch Halogen, oder ein- oder zweifach durch $(C_1$-$C_6)$Alkoxy, $(C_2$-$C_6)$Alkenyloxy, $(C_2$-$C_6)$Alkinyloxy, $(C_1$-$C_6)$Alkylthio, $(C_1$-$C_6)$Alkylsulfinyl, $(C_1$-$C_6)$Alkylsulfonyl, $(C_1$-$C_6)$-Alkoxycarbonyl oder durch Phenyl substituiert sein können; $(C_3$-$C_8)$Cycloalkyl, das ein- oder mehrfach durch Halogen, oder ein- oder zweifach durch $(C_1$-$C_4)$Alkoxy oder $(C_1$-$C_4)$Alkylthio substituiert sein kann; $(C_5$-$C_8)$Cycloalkenyl, Cyclopropylmethyl, Epoxypropyl, Furfuryl, Tetrahydrofurfuryl, Phenoxy$(C_1$-$C_6)$alkyl oder Phenyl, die beide im Phenyl-Ring durch Halogen, $(C_1$-$C_4)$Alkyl, $(C_1$-$C_4)$Alkoxy oder $NO_2$ substituiert sein können,

$R^2$ = H, $(C_1$-$C_8)$Alkyl, $(C_2$-$C_8)$Alkenyl, $(C_2$-$C_8)$Alkinyl oder $(C_1$-$C_4)$Alkoxy,

$R^3$ = einen Rest der Formel

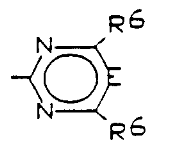 , 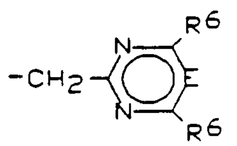 , 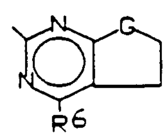 ,

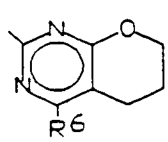 , 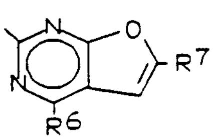 , 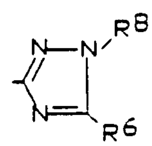 ,

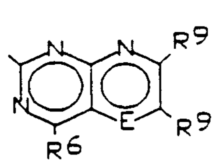 oder 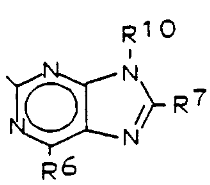

$R^4$ = unabhängig voneinander H, $(C_1$-$C_6)$Alkyl, $(C_2$-$C_6)$Alkenyl, $(C_2$-$C_6)$Alkinyl, $(C_3$-$C_8)$Cycloalkyl, wobei die vorgenannten C-haltigen Reste ein- oder mehrfach durch Halogen oder ein- oder zweifach durch $(C_1$-$C_6)$Alkoxy, $(C_2$-$C_6)$Alkenyloxy, $(C_2$-$C_6)$Alkinyloxy, $(C_1$-$C_6)$Alkylthio, $(C_1$-$C_6)$-Alkylsulfinyl, $(C_1$-$C_6)$Alkylsulfonyl oder $(C_1$-$C_6)$Alkoxycarbonyl oder durch Phenyl, das ein- oder mehrfach durch Halogen, $(C_1$-$C_4)$Alkyl, $(C_1$-$C_4)$Alkoxy, $(C_1$-$C_4)$Alkylthio, $(C_1$-$C_4)$-Alkoxycarbonyl oder $NO_2$ substituiert sein kann, substituiert sein können, ferner einer der beiden Reste $R^4$ Phenyl, das ein- oder mehrfach durch Halogen, $(C_1$-$C_4)$Alkyl, $(C_1$-$C_4)$Alkoxy, $NO_2$ oder $CF_3$ substituiert sein kann,

$R^5$ = unabhängig voneinander H, $(C_1\text{-}C_6)$Alkyl, das ein- oder mehrfach durch Halogen oder ein- oder zweifach durch $(C_1\text{-}C_6)$Alkoxy, $(C_1\text{-}C_6)$Alkylthio, $(C_1\text{-}C_6)$Alkylsulfinyl, oder $(C_1\text{-}C_6)$-Alkoxycarbonyl substituiert sein kann oder einer der Reste $R^5$ Phenyl, das ein- oder mehrfach durch Halogen, $(C_1\text{-}C_4)$Alkyl, $(C_1\text{-}C_4)$Alkoxy, $NO_2$, $CF_3$ oder $(C_1\text{-}C_6)$Alkoxycarbonyl substituiert sein kann, oder beide Reste $R^5$ gemeinsam einen Alkylenrest $-(CH_2)_n-$,

$R^6$ = unabhängig voneinander H, Halogen, $(C_1\text{-}C_6)$Alkyl, $(C_1\text{-}C_6)$Alkoxy oder $(C_1\text{-}C_6)$Alkylthio, die ein- oder mehrfach durch Halogen oder ein- oder zweifach durch $(C_1\text{-}C_4)$Alkoxy oder $(C_1\text{-}C_4)$-Alkylthio substituiert sein können; einen Rest $N(R^{11})_2$, $(C_3\text{-}C_6)$Cycloalkyl, $-OCHR^7COOR^{11}$, $(C_3\text{-}C_5)$Alkenyl, $(C_2\text{-}C_4)$Alkinyl, $(C_3\text{-}C_5)$Alkenyloxy oder $(C_3\text{-}C_5)$Alkinyloxy,

$R^7$ = H oder $(C_1\text{-}C_4)$Alkyl,

$R^8$ = $(C_1\text{-}C_4)$Alkyl, $-CHF_2$ oder $-CH_2CF_3$,

$R^9$ = unabhängig voneinander H, $(C_1\text{-}C_4)$Alkyl, $(C_1\text{-}C_4)$Alkoxy oder Halogen,

$R^{10}$ = H, $(C_1\text{-}C_4)$Alkyl, $-CHF_2$ oder $-CH_2CF_3$,

$R^{11}$ = unabhängig voneinander H, $(C_1\text{-}C_4)$Alkyl, $(C_2\text{-}C_4)$Alkenyl oder $(C_3\text{-}C_4)$Alkinyl,

E = CH oder N,

G = $CH_2$ oder O,

X = O, S oder $NR^{12}$,

Y = O oder S,

$R^{12}$ = H, $(C_1\text{-}C_6)$Alkyl, $(C_2\text{-}C_6)$Alkenyl, $(C_2\text{-}C_6)$Alkinyl, $(C_1\text{-}C_6)$Alkoxy, oder einen Rest der Formel

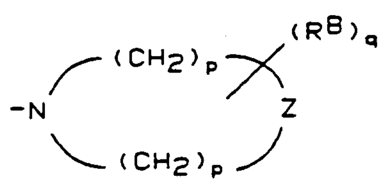

n = eine ganze Zahl von 3 bis 6,

p = eine ganze Zahl von 1 bis 3,

q = eine ganze Zahl von 0 bis 3 und

Z = O, S, $CH_2$, NH oder $N(C_1\text{-}C_4\text{-Alkyl})$ bedeuten, oder deren für die Landwirtschaft einsetzbaren Salze.

Die Verbindungen der Formel I können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, Erdalkali-, (gegebenenfalls alkylierte) Ammonium- oder organische Aminsalze. Sie werden vorzugsweise in inerten Lösungsmitteln wie z.B. Wasser, Methanol oder Aceton bei Temperaturen von 0-100 °C aus den Verbindungen der Formel I hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Bevorzugte Verbindungen der Formel I sind solche, bei denen

A einen Rest $>C(R^4)_2$ oder $>C=C(R^5)_2$,

$R^1$ $(C_1\text{-}C_4)$Alkyl, das ein- oder mehrfach durch Halogen oder ein- bis zweifach durch $(C_1\text{-}C_4)$Alkoxy substituiert sein kann, insbesondere $(C_1\text{-}C_4)$Alkyl,

$R^2$ H, $(C_1\text{-}C_4)$Alkyl oder Allyl, insbesondere H,

$R^3$ einen Rest der Formel

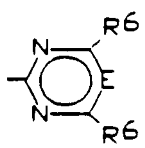

$R^4$ unabhängig voneinander H oder $(C_1\text{-}C_6)$Alkyl, das gegebenenfalls ein- oder mehrfach durch Halogen oder ein- bis zweifach durch $(C_1\text{-}C_4)$Alkoxy oder $(C_1\text{-}C_4)$Alkylthio substituiert sein kann, oder ein Rest $R^4$ $(C_2\text{-}C_6)$Alkenyl oder Phenyl, das wie oben angegeben substituiert sein kann; insbesondere steht ein Rest $R^4$ für $(C_1\text{-}C_4)$Alkyl oder Phenyl, das ein- bis dreifach durch Fluor,

Chlor, $(C_1-C_4)$Alkoxy, $(C_1-C_4)$Alkyl oder Nitro substituiert ist, und der andere Rest $R^4$ für Wasserstoff,

$R^5$ unabhängig voneinander H oder $(C_1-C_6)$Alkyl, das gegebenenfalls ein- oder mehrfach durch Halogen substituiert sein kann,

$R^6$ unabhängig voneinander Halogen, $(C_1-C_4)$Alkyl oder $(C_1-C_4)$Alkoxy, die beide halogeniert sein können, insbesondere die Reste $CH_3$, $OCH_3$, $OC_2H_5$, Cl, $OCF_2H$,

E CH oder N,

X O, S oder $NR^{12}$,

Y O oder S und

$R^{12}$ H, $(C_1-C_4)$Alkyl oder $(C_2-C_4)$Alkenyl bedeuten.

Halogen bedeutet insbesondere F, Cl oder Br. Halogeniertes $(C_1-C_4)$Alkyl oder $(C_1-C_4)$Alkoxy bedeuten insbesondere $CF_3$, $CHF_2$, $CH_2CH_2Cl$, $CH_2CH_2Br$, $CF_2CFClH$, $CH_2CF_3$, $CH_2CCl_3$, $CF_2CHF_2$, $CF_2CHFCF_3$, $CH_2CH_2CH_2Cl$, $OCF_3$, $OCF_2H$, $OCH_2CF_3$, $OCH_2CH_2Cl$.

Ein weiterer Gegenstand der vorliegenden Erfindung sind Verfahren zur Herstellung von Verbindungen der allgemeinen Formel (I)oder deren Salze, dadurch gekennzeichnet, daß man

(a) eine Verbindung der Formel (II)

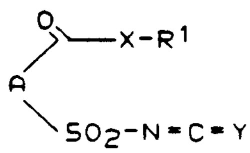

(II) ,

wobei A, X, Y, $R^1$ die oben angegebene Bedeutung haben, mit der Ausnahme, daß $R^1 \neq H$ und $X \neq NH$ sind, mit einer Verbindung der Formel (III)

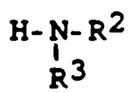

(III)

umsetzt,

(b) eine Verbindung der Formel (IV)

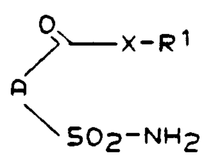

(IV)

mit einem (Thio)-Carbamat der Formel (V)

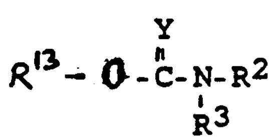

(V) ,

wobei $R^{13}$ $(C_1-C_6)$Alkyl, $(C_1-C_4)$Halogenalkyl oder Phenyl bedeutet, das ein- oder mehrfach durch Halogen, $(C_1-C_4)$Alkyl, oder $NO_2$ substituiert sein kann, umsetzt,

(c) ein (Thio)-Carbamat der Formel (VI) mit einer Verbindung der Formel (III)

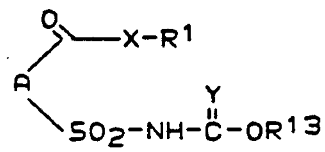

(VI),

wobei $R^{13}$ die oben angegebene Bedeutung hat, umsetzt oder
(d) eine Carbonsäure der Formel (VII)

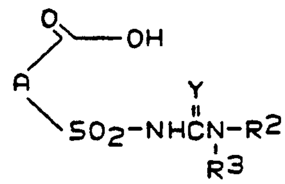

(VII)

mit einem Alkylierungsreagens der Formel (VIII)

$R^1$-X (VIII)

wobei X für eine nucleofuge Abgangsgruppe wie z.B. Halogen, Alkyl-$SO_2$-O- oder Tosyl steht, umsetzt und die erhaltenen Verbindungen gegebenenfalls in ihre Salze überführt.

Die Umsetzung der Verbindungen der Formeln (II) und (III) erfolgt vorzugsweise in inerten aprotischen Lösungsmitteln, wie z.B. Acetonitril, Dichlormethan, Toluol, Tetrahydrofuran oder Dioxan bei Temperaturen zwischen 0 °C und der Siedetemperatur des Lösungsmittels.

Die Alkylsulfonyliso-(thio)-cyanate der Formel (II) lassen sich nach im Prinzip bekannten Verfahren aus den entsprechenden Sulfonamiden der Formel (IX) in einfacher Weise herstellen

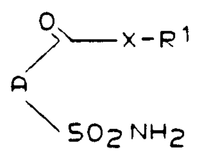

(IX)

(vgl. z.B. EP-A 085,276).

Die Sulfonamide der Formel (IX) sind bekannt oder lassen sich nach im Prinzip bekannten Verfahren herstellen, z.B. durch Umsetzung aliphatischer α-Chlorsulfonylcarbonsäureester mit Ammoniak (Bull. Soc. Chim. France 1975, 807).

Die Ausgangsstoffe der Formel (III) sind bekannt oder lassen sich nach im Prinzip bekannten Verfahren herstellen, z.B. durch Cyclisierung entsprechender Guanidinderivate mit entsprechend substituierten 1,3-Diketonen, vergleiche z.B. "The Chemistry of Heterocyclic Compounds", Vol. XVI (1962) and Supplement I (1970), oder durch Derivatisierung von Cyanurchlorid, vgl. z.B. "The Chemistry of Heterocyclic Compounds", L. Rapaport: "s-Triazines and Derivates" (1959).

Die Umsetzung der Verbindung (IV) mit den heterocyclischen Carbamaten der Formel (V) wird vorzugsweise in Gegenwart von tertiären organischen Basen wie z.B. 1,8-Diazabicyclo-5,4,0-undec-7-en (DBU) in inerten Lösungsmitteln wie Acetonitril oder Dioxan bei Temperaturen zwischen 20 °C und der Siedetemperatur des Lösungsmittels durchgeführt (analog EP-A 44 807).

Die hierzu erforderlichen Carbamate (V) sind literaturbekannt oder werden nach bekannten Verfahren hergestellt (EP A 70 804).

Die Carbamate der Formel (VI) sind neu und lassen sich durch Umsetzung der Verbindungen der Formel (IX) mit entsprechenden Chlorameisensäureestern herstellen (s. EP-A 87 780).

Die Umsetzung der (Thio)-Carbamate (VI) mit den Aminoheterocyclen führt man vorzugsweise in inerten Lösungsmitteln wie z.B. Toluol, Xylol, Chlorbenzol, Dioxan oder Acetonitril bei Temperaturen zwischen 20

°C und der Siedetemperatur des betreffenden Lösungsmittels durch.

Die Umsetzung der Carbonsäuren (VII) mit den Reagentien der Formel (VIII) wird in inerten Lösungsmitteln wie z.B. Dimethylformamid oder Dimethylsulfoxid, vorzugsweise in Gegenwart einer Hilfsbase wie z.B. Triethylamin oder Tetramethylammoniumhydroxidpentahydrat, bei Temperaturen zwischen 0 °C und der Siedetemperatur des Lösungsmittels durchgeführt.

Die Sulfonylharnstoffe der Formel I, welche im aliphatischen Rest A ein oder mehrere asymmetrische Kohlenstoffatome enthalten, liegen in enantiomeren oder diastereomeren Formen vor. Im allgemeinen werden die entsprechenden erfindungsgemäßen Verbindungen als Racemate oder als Diastereomerengemische erhalten. Falls gewünscht, können die üblichen Techniken zur Trennung dieser Gemische in die sterisch einheitlichen Bestandteile angewendet werden. Auch durch Verwendung von sterisch einheitlichen Ausgangsmaterialien ist eine Reindarstellung der genannten Verbindungen möglich.

Ferner können Sulfonylharnstoffe der Formel I, welche im aliphatischen Rest A eine oder mehrere Doppelbindungen enthalten, bei entsprechender olefinischer Substitution als E- oder als Z-Isomere vorkommen, deren Reindarstellung oder Trennung ebenfalls möglich ist. Werden z.B. ungesättigte Sulfonylisocyanate der allgemeinen Formel II als E- oder Z-Isomere eingesetzt, so werden die ungesättigten Sulfonylharnstoffe der Formel I in sterisch einheitlicher Form erhalten.

Die Formel I umfaßt daher alle obengenannten enantiomeren und diastereomeren Formen der oben definierten Verbindungen.

Die erfindungsgemäßen Verbindungen weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrauftflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt. Bei dikotylen Unkrautarten erstreckt sich das Wirkungsspektrum auf Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den perennierenden Unkräutern.

Unter den spezifischen Kulturbedingungen im Reis vorkommende Unkräuter wie z.B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Gegenstand der Erfindung ist deshalb auch ein Verfahren zur Bekämpfung von Schadpflanzen, dadurch gekennzeichnet, daß man auf diese oder die landwirtschaftlich oder industriell genutzten Böden eine wirksame Menge einer erfindungsgemäßen Verbindung der Formel I oder deren Stereoisomeren oder deren Salz appliziert.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert

oder völlig verhindert werden kann.

Gegenstand der Erfindung ist deshalb auch ein Verfahren zur Wachstumsregulation von Nutzpflanzen, dadurch gekennzeichnet, daß man auf diese oder die Anbaufläche eine wirksame Menge einer erfindungsgemäßen Verbindung der Formel I oder deren Stereoisomeren oder deren Salz appliziert. Gegenstand der Erfindung sind auch herbizide oder pflanzenwachstumsregulatorische Mittel, dadurch gekennzeichnet, daß sie eine wirksame Menge einer Verbindung der Formel I oder deren Stereoisomer oder deren Salz und inerte Hilfsstoffe enthalten. Die erfindungsgemäßen Mittel können als Spritzpulver, emulgierbare Konzentrate, Emulsionen, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2'-dinaphthylmethan-6,6'disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten, mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Diese oben genannten Formulierungstypen werden beispielsweise beschrieben in:
Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die für diese Formulierungen zu verwendenden Formulierungshilfsmittel (Inertmaterialien, Emulgatoren, Netzmittel, Tenside, Lösungsmittel etc.) sind beispielsweise in Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood oder "Encyclopedia of Surface Active Agents", Chem. Publ. Co. Inc., N.Y. 1964 beschrieben.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

**Chemische Beispiele**

Beispiel 1:

D,L-N-[4,6-Dimethoxy-pyrimidin-2-yl)aminocarbonyl]-(1-ethoxycarbonyl-eth-1-yl)sulfonamid

8,54 g (0,03 Mol) O-phenyl-N-(4,6-dimethoxy-pyrimidin-2-yl)-carbamat und 5,44 g (0,03 Mol) D,L-Ethyl-2-aminosulfonylpropanoat (hergestellt nach J. Org. Chem. 43, S. 4535 (1978)) werden in 200 ml Acetonitril suspendiert und bei 20 °C mit 5,02 g (0,033 Mol) DBU (DBU = 1,8-Diazabicyclo-5,4,0-undec-7-en) versetzt. Nach 1 h Rühren bei 20 °C wird das Reaktionsgemisch auf ca. 50 ml eingeengt, mit 200 ml Eiswasser versetzt und mit 2n HCl ein pH-Wert von ca. 3,5-4,5 eingestellt. Nach Absaugen des Reaktionsproduktes und Umkristallisieren aus Essigester/n-Hexan erhält man 10,2 g (94 % d. Th.) D,L-N-[4,6-Dimethoxy-pyrimidin-2-yl)-aminocarbonyl]-(1-ethoxycarbonyl-eth-1-yl)sulfonamid vom Schmelzpunkt 131-133 °C.

Beispiel 2:

D-N-[4,6-Dimethyl-pyrimidin-2-yl)aminocarbonyl]-(1-ethoxycarbonyl-eth-1-yl)sulfonamid

a) D-2-(tert. Butylaminosulfonyl)propionsäureethylester

22,8 g (0,2 mol) Benzylmercaptan werden in 500 ml wasserfreien Acetonitril gelöst und bei 0 °C mit 9,6 g (0,2 mol) NaH (50 %ig) langsam versetzt. Anschließend wird 2 h bei 25 °C nachgerührt und bei 0 °C eine Lösung von 21,7 g (0,2 mol) L-2-Chlorpropionsäureethylester in 100 ml Acetonitril zugetropft. Nach 1 h Reaktion bei Raumtemperatur und 2 h bei 60 °C wird das Reaktionsgemisch filtriert, das Lösungsmittel im Vakuum abdestilliert, und das resultierende Öl bei 0 °C in 250 ml Eisessig/200 ml $H_2O$ suspendiert. Anschließend wird das Gemisch bei 0 °C mit Chlor gesättigt, mit Eiswasser verdünnt, mit $CH_2Cl_2$ extrahiert und die organischen Extrakte über $Na_2SO_4$ getrocknet. Nach Abdestillieren des Lösungsmittels im Vakuum erhält man ein viskoses Öl, welches in $CH_2Cl_2$ gelöst und mit 29,2 g (0,4 mol) tert. Butylamin bei 0 °C umgesetzt wird. Nach 1 h Reaktion bei Raumtemperatur wird mit Eiswasser verdünnt und anschließend das Sulfonamid mit $CH_2Cl_2$ extrahiert. Nach Trocknen über $Na_2SO_4$ und Eindampfen des Lösungsmittels wird der Rückstand im Hochvakuum destilliert. Man erhält 37,9 g (85 %) an D-2-(tert. Butylaminosulfonyl)-propionsäureethylester vom Schmp. 75-76 °C (ee ca. 92 %).

b) D-2-(Aminosulfonyl)propionsäureethylester

23,7 g (0,1 Mol) D-2-(tert.Butylaminosulfonyl)propionsäureethylester (Beispiel 2a) werden zu 100 ml Trifluoressigsäure gegeben und ca. 1 h unter Rückfluß erhitzt. Anschließend wird im Hochvakuum eingeengt und der Rückstand aus Isopropanol umkristallisiert. Man erhält 17,5 g (97 % d. Th.) an D-2-(Aminosulfonyl)propionsäureethylester vom Schmp. 72-74 °C.

c) D-2-[(n-Butylaminocarbonyl)aminosulfonyl]propionsäure

ethylester 18,13 g (0,1 Mol) D-2-(Aminosulfonyl)propionsäureethylester wurden in 300 ml Aceton gelöst und nach Zugabe von 10,59 g (0,12 Mol) $K_2CO_3$ 30 min bei 50 °C gerührt. Anschließend werden bei

Raumtemperatur 11,9 g (0,1 Mol) n-Butylisocyanat zugetropft; die Suspension wird 5 h bei 50 °C gerührt. Nach dem Abdestillieren des Lösungsmittels wird der verbleibende Rückstand in Wasser gelöst, filtriert und das Filtrat bei 0 °C angesäuert. Nach Absaugen und Trocknen erhält man 23,8 g (85 % d. Th.) an D-2-[(n-Butylaminocarbonyl)aminosulfonyl]propionsäureethylester vom Schmp. 66-68 °C.

d) D-(1-Ethoxycarbonyl-eth-1-yl)sulfonylisocyanat

19,63 g (0,07 Mol) D-2-[(n-Butylaminocarbonyl)aminosulfonyl]propionsäureethylester von Beispiel 2c werden in 150 ml Xylol suspendiert; anschließend werden bei 130 °C ca. 2 h Phosgen eingeleitet. Nach dem Abdestillieren des Lösungsmittels erhält man das gewünschte Sulfonylisocyanat in quantitativer Ausbeute (14,5 g), IR: NCO-Bande bei 2323 $cm^{-1}$).

e) D-N-[(4,6-Dimethoxy-pyrimidin-2-yl)aminocarbonyl]-(1-ethoxycarbonyl-eth-1-yl)sulfonamid

3,1 g (0,02 Mol) 2-Amino-4,6-dimethoxy-pyrimidin werden in 50 ml $CH_2Cl_2$ gelöst und bei 0 °C mit 4,67 g (0,0225 Mol) D-(1-Ethoxycarbonyl-eth-1-yl)sulfonylisocyanat versetzt. Nach 2 h Rühren bei Raumtemperatur wird das Lösungsmittel abdestilliert und das zurückbleibende Produkt aus Essigester/n-Hexan umkristallisiert. Man erhält 6,92 g (96 % d. Th.) an D-N-[(4,6-Dimethoxy-pyrimidin-2-yl)aminocarbonyl]-(1-ethoxycarbonyl-eth-1-yl)sulfonamid vom Schmp. 134-136 °C.

Beispiel 3:

2-{N-[N-4,6-Dimethoxy-pyrimidin-2-yl)-aminocarbonyl]-aminosulfonyl}-3-phenyl-prop-2-en-säureethylester

a) 3-Phenyl-2-(tert.butylaminosulfonyl)-prop-2-en-säureethylester

11,2 g (0,05 Mol) 2-(tert.Butylaminosulfonyl)essigsäureethylester (s. J. Org. Chem. 43, S. 4535 (1978)) werden in 50 ml Benzaldehyd gelöst, mit 2 g Ammoniumacetat und 2 ml Eisessig versetzt und 2 h bei 60 °C im Wasserstrahlvakuum (20 mm) gerührt. Anschließend wird der überschüssige Aldehyd abdestilliert, der Rückstand mit n-Hexan digeriert und aus Isopropanol umkristallisiert. Man erhält 11,2 g (72 % d. Th.) 3-Phenyl-2-(tert.butylaminosulfonyl)-prop-2-en-säureethylester vom Schmp. 111-113 °C.

b) 3-Phenyl-2-aminosulfonyl-prop-2-en-säureethylester

6,23 g (0,02 Mol) 3-Phenyl-2-(tert.butylaminosulfonyl)-prop-2-en-säureethylester (Beispiel 3a) werden in 50 ml Trifluoressigsäure 1 h erhitzt. Nach Aufarbeitung analog Beispiel 2b) erhält man 4,1 g 3-Phenyl-2-aminosulfonyl-prop-2-en-säureethylester vom Schmp. 88-89 °C.

c) 2-{N-[N-(4,6-Dimethoxy-pyrimidin-2-yl)-aminocarbonyl]-aminosulfonyl}-3-phenyl-prop-2-en-säureethylester

5,10 g (0,02 Mol) (3-Phenyl-2-aminosulfonyl)-prop-2-en-carbonsäureethylester von Beispiel 3b) werden analog Beispiel 1 mit 0-Phenyl-N-(4,6-dimethoxy-pyrimidin-2-yl)-carbamat umgesetzt. Man erhält 7,41 g ( 85 % d. Th.) an 2-{N-[N-(4,6-Dimethoxy-pyrimidin-2-yl)-aminocarbonyl]-aminosulfonyl}-3-phenyl-prop-2-en-säureethylester vom Schmp. 140-143 °C.

Analog der in Beispiel 1 bis 3 beschriebenen Verfahren werden die in Tabelle 1 bis 3 aufgeführten Verbindungen hergestellt.

**Tabelle 1** (Y = O, $R^3$ = 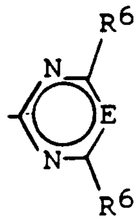 )

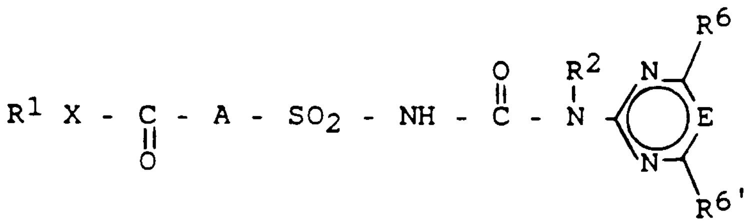

| Bsp. Nr. | A | $XR^1$ | $R^2$ | $R^6$ | $R^{6'}$ | E | Fp [°C] |
|---|---|---|---|---|---|---|---|
| 4 | $CH_2$ | $OCH_3$ | H | $CH_3$ | $CH_3$ | CH | 164-166 |
| 5 | $CH_2$ | $OCH_3$ | H | $OCH_3$ | $CH_3$ | CH | |
| 6 | $CH_2$ | $OCH_3$ | H | $OCH_3$ | $OCH_3$ | CH | 142-143 |
| 7 | $CH_2$ | $OCH_3$ | H | $OCH_3$ | Cl | CH | |
| 8 | $CH_2$ | OH | H | $CH_3$ | $CH_3$ | CH | 178-180 |
| 9 | $CH_2$ | $OCH_3$ | H | $OCHF_2$ | $OCH_3$ | CH | |
| 10 | $CH_2$ | $OCH_3$ | H | $OCHF_2$ | $OCHF_2$ | CH | |
| 11 | $CH_2$ | $OCH_3$ | H | $OCH_2CF_3$ | $OCH_3$ | CH | |
| 12 | $CH_2$ | $OCH_3$ | H | $CH_3$ | Cl | CH | |
| 13 | $CH_2$ | $OCH_3$ | H | $CH_3$ | $CH_3$ | N | |
| 14 | $CH_2$ | $OCH_3$ | H | $OCH_3$ | $CH_3$ | N | 118-119 |
| 15 | $CH_2$ | $OCH_3$ | H | $OCH_3$ | $OCH_3$ | N | |
| 16 | $CH_2$ | $OC_2H_5$ | H | $CH_3$ | $CH_3$ | CH | 170 |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 16 | $CH_2$ | $OC_2H_5$ | H | $CH_3$ | $CH_3$ | CH | 170 |
| 17 | $CH_2$ | $OC_2H_5$ | H | $OCH_3$ | $CH_3$ | CH | |
| 18 | $CH_2$ | $OC_2H_5$ | H | $OCH_3$ | $OCH_3$ | CH | |
| 19 | $CH_2$ | $OC_2H_5$ | $CH_3$ | $OCH_3$ | $OCH_3$ | CH | |
| 20 | $CH_2$ | $OC_2H_5$ | H | $OCH_3$ | Cl | CH | |
| 21 | $CH_2$ | $OC_2H_5$ | H | $OCH_3$ | $NHCH_3$ | CH | |
| 22 | $CH_2$ | $OC_2H_5$ | H | $OCH_3$ | Br | CH | |
| 23 | $CH_2$ | $OC_2H_5$ | H | $OCH_3$ | $SCH_3$ | CH | |
| 24 | $CH_2$ | $OC_2H_5$ | H | $OCHF_2$ | $OCH_3$ | CH | |
| 25 | $CH_2$ | $OC_2H_5$ | H | $OCH_3$ | $CH_3$ | N | |
| 26 | $CH_2$ | $OC_2H_5$ | H | $OC_2H_5$ | $NHCH_3$ | N | |
| 27 | $CH_2$ | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 28 | $CH_3$-CH | $OCH_3$ | H | $CH_3$ | $CH_3$ | CH | 136-138 |
| 29 | $CH_3$-CH | $OCH_3$ | H | $OCH_3$ | $CH_3$ | CH | |
| 30 | $CH_3$-CH | $OCH_3$ | H | $OCH_3$ | $OCH_3$ | CH | |
| 31 | $CH_3$-CH | $OCH_3$ | $CH_3$ | $OCH_3$ | $CH_3$ | CH | |
| 32 | $CH_3$-CH | $OCH_3$ | H | $OCH_3$ | Cl | CH | 128-130 |
| 33 | $CH_3$-CH | $OCH_3$ | H | $OCHF_2$ | $OCHF_2$ | CH | |
| 34 | $CH_3$-CH | $OCH_3$ | H | $SCH_3$ | $CH_3$ | CH | |
| 35 | $CH_3$-CH | $OCH_3$ | H | $CH(OCH_3)_2$ | $OCH_3$ | CH | |
| 36 | $CH_3$-CH | $OCH_3$ | H | $OCH_3$ | $CH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 37 | $CH_3$-CH | $OCH_3$ | H | $OCH_3$ | $OCH_3$ | N | |
| 38 | $CH_3$-CH | $OCH_3$ | H | $NH_2$ | $OCH_3$ | N | |
| 39 | $CH_3$-CH | $OCH_3$ | $CH_3$ | $OCH_3$ | $CH_3$ | N | |
| 40 (L) | $CH_3$-CH | $OCH_3$ | H | $OCH_3$ | $OCH_3$ | CH | 128-132 |
| 41 (L) | $CH_3$-CH | $OCH_3$ | H | $OCH_3$ | $CH_3$ | CH | |
| 42 (L) | $CH_3$-CH | $OCH_3$ | H | $CH_3$ | $CH_3$ | CH | |
| 43 (L) | $CH_3$-CH | $OCH_3$ | H | $OCH_3$ | $CH_3$ | N | |
| 44 (D) | $CH_3$-CH | $OC_2H_5$ | $CH_3$ | $OCH_3$ | $OCH_3$ | CH | |
| 45 (D) | $CH_3$-CH | $OC_2H_5$ | H | $OCH_3$ | Cl | CH | |

(L) bedeutet: L-Isomeres

(D) bedeutet: D-Isomeres

Fortsetzung der Tabelle 1

| Bsp. Nr. | A | $XR^1$ | $R^2$ | $R^6$ | $R^{6'}$ | E | Fp |
|---|---|---|---|---|---|---|---|
| 46 (D) | $CH_3$-CH | n-$C_3H_7$ | H | $OCH_3$ | Cl | CH | 148-149 |
| 47 | $C_2H_5$-CH | $OCH_3$ | H | $CH_3$ | $CH_3$ | CH | |
| 48 | $C_2H_5$-CH | $OCH_3$ | H | $CH_3$ | $OCH_3$ | CH | |
| 49 | $C_2H_5$-CH | $OCH_3$ | H | $OCH_3$ | $OCH_3$ | CH | 138-142 |
| 50 | $C_2H_5$-CH | $OCH_3$ | H | $OCHF_2$ | $OCHF_2$ | CH | |
| 51 | $C_2H_5$-CH | $OCH_3$ | H | $OCHF_2$ | $OCH_3$ | CH | |
| 52 | $C_2H_5$-CH | $OCH_3$ | H | $OCH_3$ | $OCH_2CF_3$ | CH | |
| 53 | $C_2H_5$-CH | $OCH_3$ | H | $OC_2H_5$ | $OCH_2CF_3$ | CH | |
| 54 | $C_2H_5$-CH | $OCH_3$ | H | $OC_2H_5$ | $OC_2H_5$ | CH | |
| 55 | $C_2H_5$-CH | $OCH_3$ | H | $SCH_3$ | $CH_3$ | CH | |
| 56 | $C_2H_5$-CH | $OCH_3$ | H | $OCH_3$ | $NHCH_3$ | CH | |
| 57 | $C_2H_5$-CH | $OCH_3$ | H | $CH_3$ | $N(CH_3)_2$ | CH | |

Fortsetzung der Tabelle 1

| Bsp. Nr. | A | $XR^1$ | $R^2$ | $R^6$ | $R^{6'}$ | E | Fp |
|---|---|---|---|---|---|---|---|
| 58 | $C_2H_5$-CH | $OCH_3$ | H | $OCH_3$ | | CH | |
| 59 | $C_2H_5$-CH | $OCH_3$ | H | $CH_3$ | | CH | |
| 60 | $C_2H_5$-CH | $OCH_3$ | H | $OCHF_2$ | $OCHF_2$ | CH | |
| 61 | $C_2H_5$-CH | $OCH_3$ | $CH_3$ | $OCH_3$ | $OCH_3$ | CH | |
| 62 | $C_2H_5$-CH | $OCH_3$ | $CH_3$ | $CH_3$ | $CH_3$ | CH | |
| 63 | $C_2H_5$-CH | $OCH_3$ | H | $OCH_3$ | $OCH_3$ | N | |
| 64 | $C_2H_5$-CH | $OCH_3$ | H | $OCH_3$ | $CH_3$ | N | 118 |
| 65 | $C_2H_5$-CH | $OCH_3$ | H | $OCH_3$ | $SCH_2$ | N | |
| 66 | $C_2H_5$-CH | $OCH_3$ | H | $CH_3$ | $CH_3$ | N | |
| 67 | $C_2H_5$-CH | $OCH_3$ | H | $OC_2H_5$ | $OCH_3$ | N | |
| 68 | $C_2H_5$-CH | $OCH_3$ | H | $OCH_3$ | $NHCH_3$ | N | |
| 69 | $C_2H_5$-CH | $OCH_3$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 70 | $C_2H_5$-CH | $OCH_3$ | H | $OCH_3$ | $NHC_2H_5$ | N | |
| 71 | $C_2H_5$-CH | $OCH_3$ | H | $OC_2H_5$ | $NHC_2H_5$ | N | |
| 72 | $C_2H_5$-CH | $OCH_3$ | H | $OCH_3$ | $N(CH_3)_2$ | N | |
| 73 | $C_2H_5$-CH | $OCH_3$ | H | $OC_2H_5$ | $N(CH_3)_2$ | N | |
| 74 | $C_2H_5$-CH | $OCH_3$ | H | $OCH_3$ | 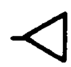 | N | |
| 75 | $C_2H_5$-CH | $OCH_3$ | H | $OCH_3$ | $OCH_2CF_3$ | N | |
| 76 | $C_2H_5$-CH | $OCH_3$ | H | $CH_3$ | $OCH_2CF_3$ | N | |
| 77 | $C_2H_5$-CH | $OCH_3$ | $CH_3$ | $OCH_3$ | $CH_3$ | N | 78-86 |
| 78 | $C_2H_5$-CH | $OCH_3$ | $CH_3$ | $OCH_3$ | $OCH_3$ | N | |
| 79 | $C_2H_5$-CH | $OCH_3$ | $CH_3$ | $OCH_3$ | $NHCH_3$ | N | |
| 80 | $C_2H_5$-CH | $OCH_3$ | H | $OCH_3$ | $OCH_3$ | CH | 48-50 |
| 81 | $C_2H_5$-CH | $OCH_3$ | H | $OCH_3$ | $CH_3$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 82 (D) | $C_2H_5$-CH | $OCH_3$ | H | $OCH_3$ | $OCH_3$ | CH | 142-145 |
| 83 (D) | $C_2H_5$-CH | $OCH_3$ | H | $CH_3$ | $OCH_3$ | CH | |
| 84 (D) | $C_2H_5$-CH | $OCH_3$ | H | $CH_3$ | $CH_3$ | CH | |
| 85 (D) | $C_2H_5$-CH | $OCH_3$ | H | $OCH_3$ | Cl | CH | |
| 86 (D) | $C_2H_5$-CH | $OCH_3$ | H | $OCHF_2$ | $OCHF_2$ | CH | |
| 87 (D) | $C_2H_5$-CH | $OCH_3$ | H | $OCHF_2$ | $OCH_3$ | CH | |
| 88 (D) | $C_2H_5$-CH | $OCH_3$ | H | $OCH_3$ | $OCH_2CF_3$ | CH | |
| 89 (D) | $C_2H_5$-CH | $OCH_3$ | H | $OC_2H_5$ | $OCH_2CF_3$ | CH | |
| 90 (D) | $C_2H_5$-CH | $OCH_3$ | H | $OC_2H_5$ | $OC_2H_5$ | CH | |
| 91 (D) | $C_2H_5$-CH | $OCH_3$ | H | $SCH_3$ | $CH_3$ | CH | |
| 92 (D) | $C_2H_5$-CH | $OCH_3$ | H | $OCH_3$ | $NHCH_3$ | CH | |
| 93 (D) | $C_2H_5$-CH | $OCH_3$ | H | $CH_3$ | $N(CH_3)_2$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 94 (D) | $C_2H_5$-CH | $OCH_3$ | H | $OCH_3$ | 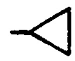 | CH | |
| 95 (D) | $C_2H_5$-CH | $OCH_3$ | H | $CH_3$ | 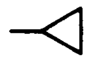 | CH | |
| 96 (D) | $C_2H_5$-CH | $OCH_3$ | H | $OCHF_2$ | $OCHF_2$ | CH | |
| 97 (D) | $C_2H_5$-CH | $OCH_3$ | $CH_3$ | $OCH_3$ | $OCH_3$ | CH | |
| 98 (D) | $C_2H_5$-CH | $OCH_3$ | $CH_3$ | $CH_3$ | $CH_3$ | CH | |
| 99 (D) | $C_2H_5$-CH | $OCH_3$ | H | $OCH_3$ | $OCH_3$ | N | |
| 100 (D) | $C_2H_5$-CH | $OCH_3$ | H | $OCH_3$ | $CH_3$ | N | |
| 101 (D) | $C_2H_5$-CH | $OCH_3$ | H | $OCH_3$ | $SCH_2$ | N | |
| 102 (D) | $C_2H_5$-CH | $OCH_3$ | H | $CH_3$ | $CH_3$ | N | |
| 103 (D) | $C_2H_5$-CH | $OCH_3$ | H | $OC_2H_5$ | $OCH_3$ | N | |
| 104 (D) | $C_2H_5$-CH | $OCH_3$ | H | $OCH_3$ | $NHCH_3$ | N | |
| 105 (D) | $C_2H_5$-CH | $OCH_3$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 106 (D) | $C_2H_5$-CH | $OCH_3$ | H | $OCH_3$ | $NHC_2H_5$ | N | |
| 107 (D) | $C_2H_5$-CH | $OCH_3$ | H | $OC_2H_5$ | $NHC_2H_5$ | N | |
| 108 (D) | $C_2H_5$-CH | $OCH_3$ | H | $OCH_3$ | $N(CH_3)_2$ | N | |
| 109 (D) | $C_2H_5$-CH | $OCH_3$ | H | $OC_2H_5$ | $N(CH_3)_2$ | N | |
| 110 (D) | $C_2H_5$-CH | $OCH_3$ | H | $OCH_3$ | 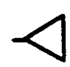 | N | |
| 111 (D) | $C_2H_5$-CH | $OCH_3$ | H | $OCH_3$ | $OCH_2CF_3$ | N | |
| 112 (D) | $C_2H_5$-CH | $OCH_3$ | H | $CH_3$ | $OCH_2CF_3$ | N | |
| 113 (D) | $C_2H_5$-CH | $OCH_3$ | $CH_3$ | $OCH_3$ | $CH_3$ | N | |
| 114 (D) | $C_2H_5$-CH | $OCH_3$ | $CH_3$ | $OCH_3$ | $OCH_3$ | N | |
| 115 (D) | $C_2H_5$-CH | $OCH_3$ | $CH_3$ | $OCH_3$ | $NHCH_3$ | N | |
| 116 (D) | $C_2H_5$-CH | $OCH_3$ | H | $OCH_3$ | $OCH_3$ | CH | |
| 117 (D) | $C_2H_5$-CH | $OCH_3$ | H | $OCH_3$ | $CH_3$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 118 (L) | $C_2H_5$-CH | $OCH_3$ | H | $OCH_3$ | $OCH_3$ | CH | |
| 119 (L) | $C_2H_5$-CH | $OCH_3$ | H | $CH_3$ | $OCH_3$ | CH | |
| 120 (L) | $C_2H_5$-CH | $OCH_3$ | H | $CH_3$ | $CH_3$ | CH | |
| 121 (L) | $C_2H_5$-CH | $OCH_3$ | H | $OCH_3$ | Cl | CH | |
| 122 (L) | $C_2H_5$-CH | $OCH_3$ | H | $OCHF_2$ | $OCHF_2$ | CH | |
| 123 (L) | $C_2H_5$-CH | $OCH_3$ | H | $OCHF_2$ | $OCH_3$ | CH | |
| 124 (L) | $C_2H_5$-CH | $OCH_3$ | H | $OCH_3$ | $OCH_2CF_3$ | CH | |
| 125 (L) | $C_2H_5$-CH | $OCH_3$ | H | $OC_2H_5$ | $OCH_2CF_3$ | CH | |
| 126 (L) | $C_2H_5$-CH | $OCH_3$ | H | $OC_2H_5$ | $OC_2H_5$ | CH | |
| 127 (L) | $C_2H_5$-CH | $OCH_3$ | H | $SCH_3$ | $CH_3$ | CH | |
| 128 (L) | $C_2H_5$-CH | $OCH_3$ | H | $OCH_3$ | $NHCH_3$ | CH | |
| 129 (L) | $C_2H_5$-CH | $OCH_3$ | H | $CH_3$ | $N(CH_3)_2$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 130 (L) | $C_2H_5$-CH | $OCH_3$ | H | $OCH_3$ | | CH | |
| 131 (L) | $C_2H_5$-CH | $OCH_3$ | H | $CH_3$ | | CH | |
| 132 (L) | $C_2H_5$-CH | $OCH_3$ | H | $OCHF_2$ | $OCHF_2$ | CH | |
| 133 (L) | $C_2H_5$-CH | $OCH_3$ | $CH_3$ | $OCH_3$ | $OCH_3$ | CH | |
| 134 (L) | $C_2H_5$-CH | $OCH_3$ | $CH_3$ | $CH_3$ | $CH_3$ | CH | |
| 135 (L) | $C_2H_5$-CH | $OCH_3$ | H | $OCH_3$ | $OCH_3$ | N | |
| 136 (L) | $C_2H_5$-CH | $OCH_3$ | H | $OCH_3$ | $CH_3$ | N | 142-144 |
| 137 (L) | $C_2H_5$-CH | $OCH_3$ | H | $OCH_3$ | $SCH_2$ | N | |
| 138 (L) | $C_2H_5$-CH | $OCH_3$ | H | $CH_3$ | $CH_3$ | N | |
| 139 (L) | $C_2H_5$-CH | $OCH_3$ | H | $OC_2H_5$ | $OCH_3$ | N | |
| 140 (L) | $C_2H_5$-CH | $OCH_3$ | H | $OCH_3$ | $NHCH_3$ | N | |
| 141 (L) | $C_2H_5$-CH | $OCH_3$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 142 (L) | $C_2H_5$-CH | $OCH_3$ | H | $OCH_3$ | $NHC_2H_5$ | N | |
| 143 (L) | $C_2H_5$-CH | $OCH_3$ | H | $OC_2H_5$ | $NHC_2H_5$ | N | |
| 144 (L) | $C_2H_5$-CH | $OCH_3$ | H | $OCH_3$ | $N(CH_3)_2$ | N | |
| 145 (L) | $C_2H_5$-CH | $OCH_3$ | H | $OC_2H_5$ | $N(CH_3)_2$ | N | |
| 146 (L) | $C_2H_5$-CH | $OCH_3$ | H | $OCH_3$ | 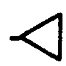 | N | |
| 147 (L) | $C_2H_5$-CH | $OCH_3$ | H | $OCH_3$ | $OCH_2CF_3$ | N | |
| 148 (L) | $C_2H_5$-CH | $OCH_3$ | H | $CH_3$ | $OCH_2CF_3$ | N | |
| 149 (L) | $C_2H_5$-CH | $OCH_3$ | $CH_3$ | $OCH_3$ | $CH_3$ | N | |
| 150 (L) | $C_2H_5$-CH | $OCH_3$ | $CH_3$ | $OCH_3$ | $OCH_3$ | N | |
| 151 (L) | $C_2H_5$-CH | $OCH_3$ | $CH_3$ | $OCH_3$ | $NHCH_3$ | N | |
| 152 (L) | $C_2H_5$-CH | $OCH_3$ | H | $OCH_3$ | $OCH_3$ | CH | |
| 153 (L) | $C_2H_5$-CH | $OCH_3$ | H | $OCH_3$ | $CH_3$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 154 | $C_2H_5$-CH | $OC_2H_5$ | H | $OCH_3$ | $OCH_3$ | CH | 102-104 |
| 155 | $C_2H_5$-CH | $OC_2H_5$ | H | $CH_3$ | $OCH_3$ | CH | |
| 156 | $C_2H_5$-CH | $OC_2H_5$ | H | $CH_3$ | $CH_3$ | CH | |
| 157 | $C_2H_5$-CH | $OC_2H_5$ | H | $OCH_3$ | Cl | CH | 78-83 |
| 158 | $C_2H_5$-CH | $OC_2H_5$ | H | $OCHF_2$ | $OCHF_2$ | CH | |
| 159 | $C_2H_5$-CH | $OC_2H_5$ | H | $OCHF_2$ | $OCH_3$ | CH | |
| 160 | $C_2H_5$-CH | $OC_2H_5$ | H | $OCH_3$ | $OCH_2CF_3$ | CH | |
| 161 | $C_2H_5$-CH | $OC_2H_5$ | H | $OC_2H_5$ | $OCH_2CF_3$ | CH | |
| 162 | $C_2H_5$-CH | $OC_2H_5$ | H | $OC_2H_5$ | $OC_2H_5$ | CH | |
| 163 | $C_2H_5$-CH | $OC_2H_5$ | H | $SCH_3$ | $CH_3$ | CH | |
| 164 | $C_2H_5$-CH | $OC_2H_5$ | H | $OCH_3$ | $NHCH_3$ | CH | |
| 165 | $C_2H_5$-CH | $OC_2H_5$ | H | $CH_3$ | $N(CH_3)_2$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 166 | $C_2H_5$-CH | $OC_2H_5$ | H | $OCH_3$ | 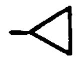 | CH | |
| 167 | $C_2H_5$-CH | $OC_2H_5$ | H | $CH_3$ | 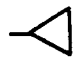 | CH | |
| 168 | $C_2H_5$-CH | $OC_2H_5$ | H | $OCHF_2$ | $OCHF_2$ | CH | |
| 169 | $C_2H_5$-CH | $OC_2H_5$ | $CH_3$ | $OCH_3$ | $OCH_3$ | CH | |
| 170 | $C_2H_5$-CH | $OC_2H_5$ | $CH_3$ | $CH_3$ | $CH_3$ | CH | |
| 171 | $C_2H_5$-CH | $OC_2H_5$ | H | $OCH_3$ | $OCH_3$ | N | |
| 172 | $C_2H_5$-CH | $OC_2H_5$ | H | $OCH_3$ | $CH_3$ | N | |
| 173 | $C_2H_5$-CH | $OC_2H_5$ | H | $OCH_3$ | $SCH_2$ | N | |
| 174 | $C_2H_5$-CH | $OC_2H_5$ | H | $CH_3$ | $CH_3$ | N | |
| 175 | $C_2H_5$-CH | $OC_2H_5$ | H | $OC_2H_5$ | $OCH_3$ | N | |
| 176 | $C_2H_5$-CH | $OC_2H_5$ | H | $OCH_3$ | $NHCH_3$ | N | |
| 177 | $C_2H_5$-CH | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 178 | $C_2H_5$-CH | $OC_2H_5$ | H | $OCH_3$ | $NHC_2H_5$ | N | |
| 179 | $C_2H_5$-CH | $OC_2H_5$ | H | $OC_2H_5$ | $NHC_2H_5$ | N | |
| 180 | $C_2H_5$-CH | $OC_2H_5$ | H | $OCH_3$ | $N(CH_3)_2$ | N | |
| 181 | $C_2H_5$-CH | $OC_2H_5$ | H | $OC_2H_5$ | $N(CH_3)_2$ | N | |
| 182 | $C_2H_5$-CH | $OC_2H_5$ | H | $OCH_3$ | 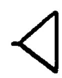 | N | |
| 183 | $C_2H_5$-CH | $OC_2H_5$ | H | $OCH_3$ | $OCH_2CF_3$ | N | |
| 184 | $C_2H_5$-CH | $OC_2H_5$ | H | $CH_3$ | $OCH_2CF_3$ | N | |
| 185 | $C_2H_5$-CH | $OC_2H_5$ | $CH_3$ | $OCH_3$ | $CH_3$ | N | |
| 186 | $C_2H_5$-CH | $OC_2H_5$ | $CH_3$ | $OCH_3$ | $OCH_3$ | N | |
| 187 | $C_2H_5$-CH | $OC_2H_5$ | $CH_3$ | $OCH_3$ | $NHCH_3$ | N | |
| 188 | $C_2H_5$-CH | $OC_2H_5$ | H | $OCH_3$ | $OCH_3$ | CH | |
| 189 | $C_2H_5$-CH | $OC_2H_5$ | H | $OCH_3$ | $CH_3$ | CH | |

Fortsetzung der Tabelle 1

| Bsp. Nr. | A | $XR^1$ | $R^2$ | $R^6$ | $R^{6'}$ | E | Fp |
|---|---|---|---|---|---|---|---|
| 190 (L) | $C_2H_5$-CH | $OC_2H_5$ | H | $OCH_3$ | $OCH_3$ | CH | |
| 191 (L) | $C_2H_5$-CH | $OC_2H_5$ | H | $CH_3$ | $OCH_3$ | CH | |
| 192 (L) | $C_2H_5$-CH | $OC_2H_5$ | H | $CH_3$ | $CH_3$ | CH | |
| 193 (L) | $C_2H_5$-CH | $OC_2H_5$ | H | $OCH_3$ | Cl | CH | |
| 194 (L) | $C_2H_5$-CH | $OC_2H_5$ | H | $OCHF_2$ | $OCHF_2$ | CH | |
| 195 (L) | $C_2H_5$-CH | $OC_2H_5$ | H | $OCHF_2$ | $OCH_3$ | CH | |
| 196 (L) | $C_2H_5$-CH | $OC_2H_5$ | H | $OCH_3$ | $OCH_2CF_3$ | CH | |
| 197 (L) | $C_2H_5$-CH | $OC_2H_5$ | H | $OC_2H_5$ | $OCH_2CF_3$ | CH | |
| 198 (L) | $C_2H_5$-CH | $OC_2H_5$ | H | $OC_2H_5$ | $OC_2H_5$ | CH | |
| 199 (L) | $C_2H_5$-CH | $OC_2H_5$ | H | $SCH_3$ | $CH_3$ | CH | |
| 200 (L) | $C_2H_5$-CH | $OC_2H_5$ | H | $OCH_3$ | $NHCH_3$ | CH | |
| 201 (L) | $C_2H_5$-CH | $OC_2H_5$ | H | $CH_3$ | $N(CH_3)_2$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 202 (L) | $C_2H_5$-CH | $OC_2H_5$ | H | $OCH_3$ | 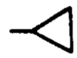 | CH | |
| 203 (L) | $C_2H_5$-CH | $OC_2H_5$ | H | $CH_3$ | 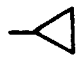 | CH | |
| 204 (L) | $C_2H_5$-CH | $OC_2H_5$ | H | $OCHF_2$ | $OCHF_2$ | CH | |
| 205 (L) | $C_2H_5$-CH | $OC_2H_5$ | $CH_3$ | $OCH_3$ | $OCH_3$ | CH | |
| 206 (L) | $C_2H_5$-CH | $OC_2H_5$ | $CH_3$ | $CH_3$ | $CH_3$ | CH | |
| 207 (L) | $C_2H_5$-CH | $OC_2H_5$ | H | $OCH_3$ | $OCH_3$ | N | |
| 208 (L) | $C_2H_5$-CH | $OC_2H_5$ | H | $OCH_3$ | $CH_3$ | N | |
| 209 (L) | $C_2H_5$-CH | $OC_2H_5$ | H | $OCH_3$ | $SCH_2$ | N | |
| 210 (L) | $C_2H_5$-CH | $OC_2H_5$ | H | $CH_3$ | $CH_3$ | N | |
| 211 (L) | $C_2H_5$-CH | $OC_2H_5$ | H | $OC_2H_5$ | $OCH_3$ | N | |
| 212 (L) | $C_2H_5$-CH | $OC_2H_5$ | H | $OCH_3$ | $NHCH_3$ | N | |
| 213 (L) | $C_2H_5$-CH | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 214 (L) | $C_2H_5$-CH | $OC_2H_5$ | H | $OCH_3$ | $NHC_2H_5$ | N | |
| 215 (L) | $C_2H_5$-CH | $OC_2H_5$ | H | $OC_2H_5$ | $NHC_2H_5$ | N | |
| 216 (L) | $C_2H_5$-CH | $OC_2H_5$ | H | $OCH_3$ | $N(CH_3)_2$ | N | |
| 217 (L) | $C_2H_5$-CH | $OC_2H_5$ | H | $OC_2H_5$ | $N(CH_3)_2$ | N | |
| 218 (L) | $C_2H_5$-CH | $OC_2H_5$ | H | $OCH_3$ | 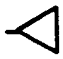 | N | |
| 219 (L) | $C_2H_5$-CH | $OC_2H_5$ | H | $OCH_3$ | $OCH_2CF_3$ | N | |
| 220 (L) | $C_2H_5$-CH | $OC_2H_5$ | H | $CH_3$ | $OCH_2CF_3$ | N | |
| 221 (L) | $C_2H_5$-CH | $OC_2H_5$ | $CH_3$ | $OCH_3$ | $CH_3$ | N | |
| 222 (L) | $C_2H_5$-CH | $OC_2H_5$ | $CH_3$ | $OCH_3$ | $OCH_3$ | N | |
| 223 (L) | $C_2H_5$-CH | $OC_2H_5$ | $CH_3$ | $OCH_3$ | $NHCH_3$ | N | |
| 224 (L) | $C_2H_5$-CH | $OC_2H_5$ | H | $OCH_3$ | $OCH_3$ | CH | |
| 225 (L) | $C_2H_5$-CH | $OC_2H_5$ | H | $OCH_3$ | $CH_3$ | CH | |

Fortsetzung der Tabelle 1

| Bsp. Nr. | A | $XR^1$ | $R^2$ | $R^6$ | $R^{6'}$ | E | Fp |
|---|---|---|---|---|---|---|---|
| 226 (D) | $C_2H_5$-CH | $OC_2H_5$ | H | $OCH_3$ | $OCH_3$ | CH | |
| 227 (D) | $C_2H_5$-CH | $OC_2H_5$ | H | $CH_3$ | $OCH_3$ | CH | |
| 228 (D) | $C_2H_5$-CH | $OC_2H_5$ | H | $CH_3$ | $CH_3$ | CH | |
| 229 (D) | $C_2H_5$-CH | $OC_2H_5$ | H | $OCH_3$ | Cl | CH | |
| 230 (D) | $C_2H_5$-CH | $OC_2H_5$ | H | $OCHF_2$ | $OCHF_2$ | CH | 182-184 |
| 231 (D) | $C_2H_5$-CH | $OC_2H_5$ | H | $OCHF_2$ | $OCH_3$ | CH | |
| 232 (D) | $C_2H_5$-CH | $OC_2H_5$ | H | $OCH_3$ | $OCH_2CF_3$ | CH | |
| 233 (D) | $C_2H_5$-CH | $OC_2H_5$ | H | $OC_2H_5$ | $OCH_2CF_3$ | CH | |
| 234 (D) | $C_2H_5$-CH | $OC_2H_5$ | H | $OC_2H_5$ | $OC_2H_5$ | CH | |
| 235 (D) | $C_2H_5$-CH | $OC_2H_5$ | H | $SCH_3$ | $CH_3$ | CH | |
| 236 (D) | $C_2H_5$-CH | $OC_2H_5$ | H | $OCH_3$ | $NHCH_3$ | CH | |
| 237 (D) | $C_2H_5$-CH | $OC_2H_5$ | H | $CH_3$ | $N(CH_3)_2$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 238 (D) | $C_2H_5$-CH | $OC_2H_5$ | H | $OCH_3$ | 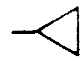 | CH | |
| 239 (D) | $C_2H_5$-CH | $OC_2H_5$ | H | $CH_3$ | 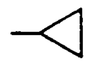 | CH | |
| 240 (D) | $C_2H_5$-CH | $OC_2H_5$ | H | $OCHF_2$ | $OCHF_2$ | CH | |
| 241 (D) | $C_2H_5$-CH | $OC_2H_5$ | $CH_3$ | $OCH_3$ | $OCH_3$ | CH | |
| 242 (D) | $C_2H_5$-CH | $OC_2H_5$ | $CH_3$ | $CH_3$ | $CH_3$ | CH | |
| 243 (D) | $C_2H_5$-CH | $OC_2H_5$ | H | $OCH_3$ | $OCH_3$ | N | |
| 244 (D) | $C_2H_5$-CH | $OC_2H_5$ | H | $OCH_3$ | $CH_3$ | N | |
| 245 (D) | $C_2H_5$-CH | $OC_2H_5$ | H | $OCH_3$ | $SCH_2$ | N | |
| 246 (D) | $C_2H_5$-CH | $OC_2H_5$ | H | $CH_3$ | $CH_3$ | N | |
| 247 (D) | $C_2H_5$-CH | $OC_2H_5$ | H | $OC_2H_5$ | $OCH_3$ | N | |
| 248 (D) | $C_2H_5$-CH | $OC_2H_5$ | H | $OCH_3$ | $NHCH_3$ | N | |
| 249 (D) | $C_2H_5$-CH | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 250 (D) | $C_2H_5$-CH | $OC_2H_5$ | H | $OCH_3$ | $NHC_2H_5$ | N | |
| 251 (D) | $C_2H_5$-CH | $OC_2H_5$ | H | $OC_2H_5$ | $NHC_2H_5$ | N | |
| 252 (D) | $C_2H_5$-CH | $OC_2H_5$ | H | $OCH_3$ | $N(CH_3)_2$ | N | |
| 253 (D) | $C_2H_5$-CH | $OC_2H_5$ | H | $OC_2H_5$ | $N(CH_3)_2$ | N | |
| 254 (D) | $C_2H_5$-CH | $OC_2H_5$ | H | $OCH_3$ | 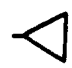 | N | |
| 255 (D) | $C_2H_5$-CH | $OC_2H_5$ | H | $OCH_3$ | $OCH_2CF_3$ | N | |
| 256 (D) | $C_2H_5$-CH | $OC_2H_5$ | H | $CH_3$ | $OCH_2CF_3$ | N | |
| 257 (D) | $C_2H_5$-CH | $OC_2H_5$ | $CH_3$ | $OCH_3$ | $CH_3$ | N | |
| 258 (D) | $C_2H_5$-CH | $OC_2H_5$ | $CH_3$ | $OCH_3$ | $OCH_3$ | N | |
| 259 (D) | $C_2H_5$-CH | $OC_2H_5$ | $CH_3$ | $OCH_3$ | $NHCH_3$ | N | |
| 260 (D) | $C_2H_5$-CH | $OC_2H_5$ | H | $OCH_3$ | $OCH_3$ | CH | |
| 261 (D) | $C_2H_5$-CH | $OC_2H_5$ | H | $OCH_3$ | $CH_3$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 262 | n-$C_3H_7$-CH | $OC_2H_5$ | H | $CH_3$ | $CH_3$ | CH | |
| 263 | n-$C_3H_7$-CH | $OC_2H_5$ | H | $OCH_3$ | $CH_3$ | CH | |
| 264 | n-$C_3H_7$-CH | $OC_2H_5$ | H | $OCH_3$ | $OCH_3$ | CH | 76-78 |
| 265 | n-$C_3H_7$-CH | $OC_2H_5$ | $CH_3$ | $OCH_3$ | $OCH_3$ | CH | |
| 266 | n-$C_3H_7$-CH | $OC_2H_5$ | H | $OCH_3$ | Cl | CH | |
| 267 | n-$C_3H_7$-CH | $OC_2H_5$ | H | $OCH_3$ | $NHCH_3$ | CH | |
| 268 | n-$C_3H_7$-CH | $OC_2H_5$ | H | $OCH_3$ | Br | CH | |
| 269 | n-$C_3H_7$-CH | $OC_2H_5$ | H | $OCH_3$ | $SCH_3$ | CH | |
| 270 | n-$C_3H_7$-CH | $OC_2H_5$ | H | $OCHF_2$ | $OCH_3$ | CH | |
| 271 | n-$C_3H_7$-CH | $OC_2H_5$ | H | $OCH_3$ | $CH_3$ | N | |
| 272 | n-$C_3H_7$-CH | $OC_2H_5$ | H | $OC_2H_5$ | $NHCH_3$ | N | |
| 273 | n-$C_3H_7$-CH | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 274 | 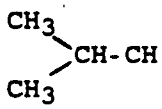 | $OC_2H_5$ | H | $CH_3$ | $CH_3$ | CH | |
| 275 | 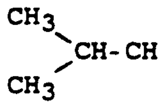 | $OC_2H_5$ | H | $OCH_3$ | $CH_3$ | CH | |
| 276 | 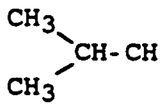 | $OC_2H_5$ | H | $OCH_3$ | $OCH_3$ | CH | 98-100 |
| 277 | 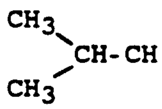 | $OC_2H_5$ | $CH_3$ | $OCH_3$ | $OCH_3$ | CH | |
| 278 | 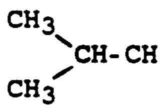 | $OC_2H_5$ | H | $OCH_3$ | Cl | CH | |
| 279 | 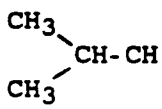 | $OC_2H_5$ | H | $OCH_3$ | $NHCH_3$ | CH | |
| 280 | 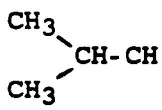 | $OC_2H_5$ | H | $OCH_3$ | Br | CH | |
| 281 | 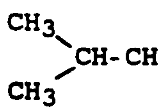 | $OC_2H_5$ | H | $OCH_3$ | $SCH_3$ | CH | |
| 282 | 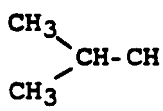 | $OC_2H_5$ | H | $OCHF_2$ | $OCH_3$ | CH | |
| 283 | 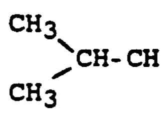 | $OC_2H_5$ | H | $OCH_3$ | $CH_3$ | N | |
| 284 | 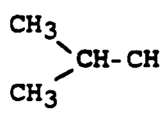 | $OC_2H_5$ | H | $OC_2H_5$ | $NHCH_3$ | N | |
| 285 | 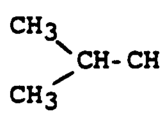 | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 286 | 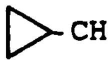 | $OCH_3$ | H | $CH_3$ | $CH_3$ | CH | |
| 287 | 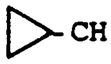 | $OCH_3$ | H | $OCH_3$ | $CH_3$ | CH | |
| 288 | 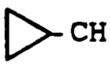 | $OCH_3$ | H | $OCH_3$ | $OCH_3$ | CH | |
| 289 | 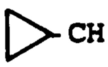 | $OCH_3$ | $CH_3$ | $OCH_3$ | $OCH_3$ | CH | |
| 290 | 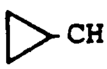 | $OCH_3$ | H | $OCH_3$ | Cl | CH | |
| 291 | 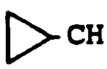 | $OCH_3$ | H | $OCH_3$ | $NHCH_3$ | CH | |
| 292 | 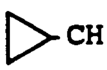 | $OCH_3$ | H | $OCH_3$ | Br | CH | |
| 293 | 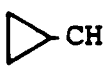 | $OCH_3$ | H | $OCH_3$ | $SCH_3$ | CH | |
| 294 | 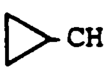 | $OCH_3$ | H | $OCHF_2$ | $OCH_3$ | CH | |
| 295 | 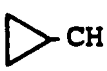 | $OCH_3$ | H | $OCH_3$ | $CH_3$ | N | |
| 296 | 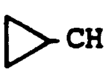 | $OCH_3$ | H | $OC_2H_5$ | $NHCH_3$ | N | |
| 297 | 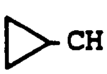 | $OCH_3$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 298 | 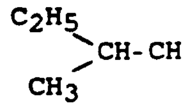 | $OC_2H_5$ | H | $CH_3$ | $CH_3$ | CH | |
| 299 | 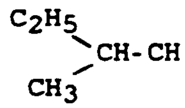 | $OC_2H_5$ | H | $OCH_3$ | $CH_3$ | CH | |
| 300 | 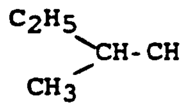 | $OC_2H_5$ | H | $OCH_3$ | $OCH_3$ | CH | Harz |
| 301 | 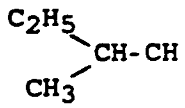 | $OC_2H_5$ | $CH_3$ | $OCH_3$ | $OCH_3$ | CH | |
| 302 | 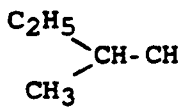 | $OC_2H_5$ | H | $OCH_3$ | Cl | CH | |
| 303 | 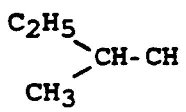 | $OC_2H_5$ | H | $OCH_3$ | $NHCH_3$ | CH | |
| 304 | 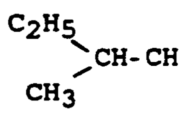 | $OC_2H_5$ | H | $OCH_3$ | Br | CH | |
| 305 | 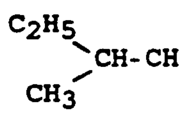 | $OC_2H_5$ | H | $OCH_3$ | $SCH_3$ | CH | |
| 306 | 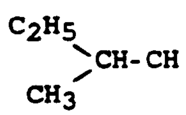 | $OC_2H_5$ | H | $OCHF_2$ | $OCH_3$ | CH | |
| 307 | 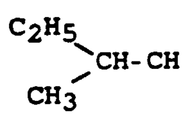 | $OC_2H_5$ | H | $OCH_3$ | $CH_3$ | N | |
| 308 | 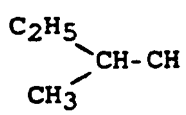 | $OC_2H_5$ | H | $OC_2H_5$ | $NHCH_3$ | N | |
| 309 | 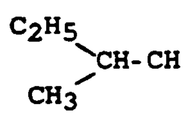 | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 310 | 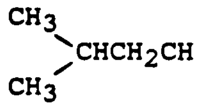 | $OC_2H_5$ | H | $CH_3$ | $CH_3$ | CH | |
| 311 | 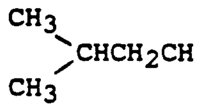 | $OC_2H_5$ | H | $OCH_3$ | $CH_3$ | CH | |
| 312 | 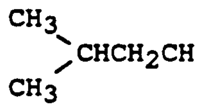 | $OC_2H_5$ | H | $OCH_3$ | $OCH_3$ | CH | |
| 313 | 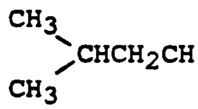 | $OC_2H_5$ | $CH_3$ | $OCH_3$ | $OCH_3$ | CH | |
| 314 | 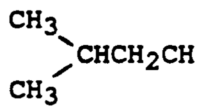 | $OC_2H_5$ | H | $OCH_3$ | Cl | CH | |
| 315 | 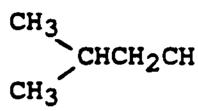 | $OC_2H_5$ | H | $OCH_3$ | $NHCH_3$ | CH | |
| 316 | 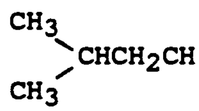 | $OC_2H_5$ | H | $OCH_3$ | Br | CH | |
| 317 | 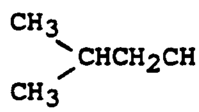 | $OC_2H_5$ | H | $OCH_3$ | $SCH_3$ | CH | |
| 318 | 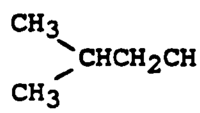 | $OC_2H_5$ | H | $OCHF_2$ | $OCH_3$ | CH | |
| 319 | 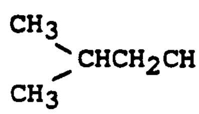 | $OC_2H_5$ | H | $OCH_3$ | $CH_3$ | N | |
| 320 | 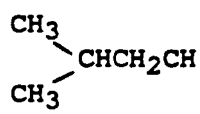 | $OC_2H_5$ | H | $OC_2H_5$ | $NHCH_3$ | N | |
| 321 | 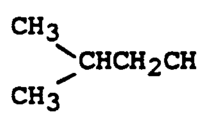 | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 322 | $CH_2$=CH-CH | $OC_2H_5$ | H | $CH_3$ | $CH_3$ | CH | |
| 323 | $CH_2$=CH-CH | $OC_2H_5$ | H | $OCH_3$ | $CH_3$ | CH | |
| 324 | $CH_2$=CH-CH | $OC_2H_5$ | H | $OCH_3$ | $OCH_3$ | CH | |
| 325 | $CH_2$=CH-CH | $OC_2H_5$ | $CH_3$ | $OCH_3$ | $OCH_3$ | CH | |
| 326 | $CH_2$=CH-CH | $OC_2H_5$ | H | $OCH_3$ | Cl | CH | |
| 327 | $CH_2$=CH-CH | $OC_2H_5$ | H | $OCH_3$ | $NHCH_3$ | CH | |
| 328 | $CH_2$=CH-CH | $OC_2H_5$ | H | $OCH_3$ | Br | CH | |
| 329 | $CH_2$=CH-CH | $OC_2H_5$ | H | $OCH_3$ | $SCH_3$ | CH | |
| 330 | $CH_2$=CH-CH | $OC_2H_5$ | H | $OCHF_2$ | $OCH_3$ | CH | |
| 331 | $CH_2$=CH-CH | $OC_2H_5$ | H | $OCH_3$ | $CH_3$ | N | |
| 332 | $CH_2$=CH-CH | $OC_2H_5$ | H | $OC_2H_5$ | $NHCH_3$ | N | |
| 333 | $CH_2$=CH-CH | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 334 | 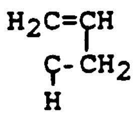 | $OC_2H_5$ | H | $CH_3$ | $CH_3$ | CH | |
| 335 | 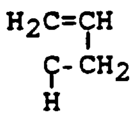 | $OC_2H_5$ | H | $OCH_3$ | $CH_3$ | CH | |
| 336 | 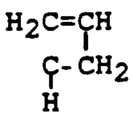 | $OC_2H_5$ | H | $OCH_3$ | $OCH_3$ | CH | 128-129 |
| 337 | 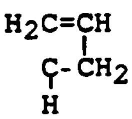 | $OC_2H_5$ | $CH_3$ | $OCH_3$ | $OCH_3$ | CH | |
| 338 | 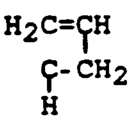 | $OC_2H_5$ | H | $OCH_3$ | Cl | CH | |
| 339 | 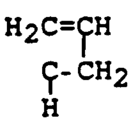 | $OC_2H_5$ | H | $OCH_3$ | $NHCH_3$ | CH | |
| 340 | 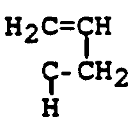 | $OC_2H_5$ | H | $OCH_3$ | Br | CH | |
| 341 | 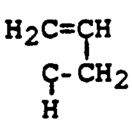 | $OC_2H_5$ | H | $OCH_3$ | $SCH_3$ | CH | |
| 342 | 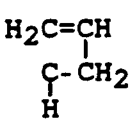 | $OC_2H_5$ | H | $OCHF_2$ | $OCH_3$ | CH | |
| 343 | 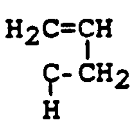 | $OC_2H_5$ | H | $OCH_3$ | $CH_3$ | N | |
| 344 | 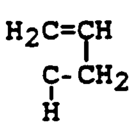 | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 345 | 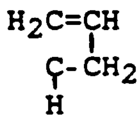 | $OC_2H_5$ | H | $OCH_3$ | $N(CH_3)_2$ | N | |
| 346 | $ClCH_2$-CH | $OC_2H_5$ | H | $CH_3$ | $CH_3$ | CH | |
| 347 | $ClCH_2$-CH | $OC_2H_5$ | H | $OCH_3$ | $CH_3$ | CH | |
| 348 | $ClCH_2$-CH | $OC_2H_5$ | H | $OCH_3$ | $OCH_3$ | CH | 68-70 |
| 349 | $ClCH_2$-CH | $OC_2H_5$ | $CH_3$ | $OCH_3$ | $OCH_3$ | CH | |
| 350 | $CF_3$-CH | $OC_2H_5$ | H | $OCH_3$ | Cl | CH | |
| 351 | $CF_3$-CH | $OC_2H_5$ | H | $OCH_3$ | $NHCH_3$ | CH | |
| 352 | $CF_3$-CH | $OC_2H_5$ | H | $OCH_3$ | Br | CH | |
| 353 | $CF_3$-CH | $OC_2H_5$ | H | $OCH_3$ | $SCH_3$ | CH | |
| 354 | $CF_3CF_2$-CH | $OC_2H_5$ | H | $OCHF_2$ | $OCH_3$ | CH | |
| 355 | $CF_3CF_2$-CH | $OC_2H_5$ | H | $OCH_3$ | $CH_3$ | N | |
| 356 | $CF_3CF_2$-CH | $OC_2H_5$ | H | $OC_2H_5$ | $NHCH_3$ | N | |
| 357 | $CF_3CF_2$-CH | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 358 | $CH_3SCH_2$-CH | $OC_2H_5$ | H | $CH_3$ | $CH_3$ | CH | |
| 359 | $CH_3SCH_2$-CH | $OC_2H_5$ | H | $OCH_3$ | $CH_3$ | CH | |
| 360 | $CH_3SCH_2$-CH | $OC_2H_5$ | H | $OCH_3$ | $OCH_3$ | CH | |
| 361 | $CH_3OCH_2$-CH | $OC_2H_5$ | $CH_3$ | $OCH_3$ | $OCH_3$ | CH | |
| 362 | $CH_3OCH_2$-CH | $OC_2H_5$ | H | $OCH_3$ | Cl | CH | |
| 363 | $CH_3OCH_2$-CH | $OC_2H_5$ | H | $OCH_3$ | $NHCH_3$ | CH | |
| 364 | $CH_3SCH_2CH_2$-CH | $OC_2H_5$ | H | $OCH_3$ | Br | CH | |
| 365 | $CH_3SCH_2CH_2$-CH | $OC_2H_5$ | H | $OCH_3$ | $SCH_3$ | CH | |
| 366 | $CH_3SCH_2CH_2$-CH | $OC_2H_5$ | H | $OCHF_2$ | $OCH_3$ | CH | |
| 367 | $CH_3OCH_2CH_2$-CH | $OC_2H_5$ | H | $OCH_3$ | $CH_3$ | N | |
| 368 | $CH_3OCH_2CH_2$-CH | $OC_2H_5$ | H | $OC_2H_5$ | $NHCH_3$ | N | |
| 369 | $ClCH_2CH_2CH$ | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 370 | $C_6H_5$-CH | $OC_2H_5$ | H | $CH_3$ | $CH_3$ | CH | |
| 371 | $C_6H_5$-CH | $OC_2H_5$ | H | $OCH_3$ | $CH_3$ | CH | |
| 372 | $C_6H_5$-CH | $OC_2H_5$ | H | $OCH_3$ | $OCH_3$ | CH | |
| 373 | $C_6H_5$-CH | $OC_2H_5$ | $CH_3$ | $OCH_3$ | $OCH_3$ | CH | |
| 374 | $C_6H_5$-CH | $OC_2H_5$ | H | $OCH_3$ | Cl | CH | |
| 375 | 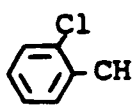 | $OC_2H_5$ | H | $OCH_3$ | $NHCH_3$ | CH | |
| 376 | " | $OC_2H_5$ | H | $OCH_3$ | Br | CH | |
| 377 | " | $OC_2H_5$ | H | $OCH_3$ | $SCH_3$ | CH | |
| 378 | 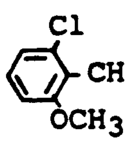 | $OC_2H_5$ | H | $OCHF_2$ | $OCH_3$ | CH | |
| 379 | 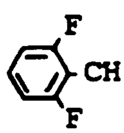 | $OCH_3$ | H | $OCH_3$ | $CH_3$ | N | |
| 380 | 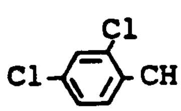 | $OCH_3$ | H | $OC_2H_5$ | $NHCH_3$ | N | |
| 381 | " | $OCH_3$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 382 | $C_6H_5-CH_2-CH$ | $OC_2H_5$ | H | $CH_3$ | $CH_3$ | CH | |
| 383 | $C_6H_5-CH_2-CH$ | $OC_2H_5$ | H | $OCH_3$ | $CH_3$ | CH | |
| 384 | $C_6H_5-CH_2-CH$ | $OC_2H_5$ | H | $OCH_3$ | $OCH_3$ | CH | 144-145 |
| 385 | $C_6H_5-CH_2-CH$ | $OC_2H_5$ | $CH_3$ | $OCH_3$ | $OCH_3$ | CH | |
| 386 | $C_6H_5-CH_2-CH$ | $OC_2H_5$ | H | $OCH_3$ | Cl | CH | |
| 387 | $C_6H_5-CH_2-CH$ | $OC_2H_5$ | H | $OCH_3$ | $NHCH_3$ | CH | |
| 388 | $C_6H_5-CH_2-CH$ | $OC_2H_5$ | H | $OCH_3$ | Br | CH | |
| 389 | $C_6H_5-CH_2-CH$ | $OC_2H_5$ | H | $OCH_3$ | $SCH_3$ | CH | |
| 390 | $C_6H_5-CH_2-CH$ | $OC_2H_5$ | H | $OCHF_2$ | $OCH_3$ | CH | |
| 391 | $C_6H_5-CH_2-CH$ | $OC_2H_5$ | H | $OCH_3$ | $CH_3$ | N | |
| 392 | $C_6H_5-CH_2-CH$ | $OC_2H_5$ | H | $OC_2H_5$ | $NHCH_3$ | N | |
| 393 | $C_6H_5-CH_2-CH$ | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 370 | 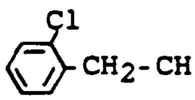 | $OC_2H_5$ | H | $CH_3$ | $CH_3$ | CH | |
| 371 | " | $OC_2H_5$ | H | $OCH_3$ | $CH_3$ | CH | |
| 372 | " | $OC_2H_5$ | H | $OCH_3$ | $OCH_3$ | CH | |
| 373 | 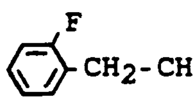 | $OC_2H_5$ | $CH_3$ | $OCH_3$ | $OCH_3$ | CH | |
| 374 | " | $OC_2H_5$ | H | $OCH_3$ | Cl | CH | |
| 375 | " | $OC_2H_5$ | H | $OCH_3$ | $NHCH_3$ | CH | |
| 376 | 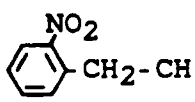 | $OC_2H_5$ | H | $OCH_3$ | Br | CH | |
| 377 | " | $OC_2H_5$ | H | $OCH_3$ | $SCH_3$ | CH | |
| 378 | " | $OC_2H_5$ | H | $OCHF_2$ | $OCH_3$ | CH | |
| 379 | 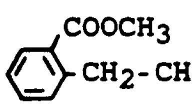 | $OCH_3$ | H | $OCH_3$ | $CH_3$ | N | |
| 380 | " | $OCH_3$ | H | $OC_2H_5$ | $NHCH_3$ | N | |
| 381 | " | $OCH_3$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 406 | $CH_2=C$ | $OCH_3$ | H | $CH_3$ | $CH_3$ | CH | |
| 407 | $CH_2=C$ | $OCH_3$ | H | $OCH_3$ | $CH_3$ | CH | |
| 408 | $CH_2=C$ | $OCH_3$ | H | $OCH_3$ | $OCH_3$ | CH | |
| 409 | $CH_2=C$ | $OCH_3$ | $CH_3$ | $OCH_3$ | $OCH_3$ | CH | |
| 410 | $CH_2=C$ | $OCH_3$ | H | $OCH_3$ | Cl | CH | |
| 411 | $CH_2=C$ | $OCH_3$ | H | $OCH_3$ | $NHCH_3$ | CH | |
| 412 | $CH_2=C$ | $OCH_3$ | H | $OCH_3$ | Br | CH | |
| 413 | $CH_2=C$ | $OCH_3$ | H | $OCH_3$ | $SCH_3$ | CH | |
| 414 | $CH_2=C$ | $OCH_3$ | H | $OCHF_2$ | $OCH_3$ | CH | |
| 415 | $CH_2=C$ | $OCH_3$ | H | $OCH_3$ | $CH_3$ | N | |
| 416 | $CH_2=C$ | $OCH_3$ | H | $OC_2H_5$ | $NHCH_3$ | N | |
| 417 | $CH_2=C$ | $OCH_3$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 418 | $CH_3CH=C$ | $OC_2H_5$ | H | $CH_3$ | $CH_3$ | CH | |
| 419 | $CH_3CH=C$ | $OC_2H_5$ | H | $OCH_3$ | $CH_3$ | CH | |
| 420 | $CH_3CH=C$ | $OC_2H_5$ | H | $OCH_3$ | $OCH_3$ | CH | 122-123 |
| 421 | $CH_3CH=C$ | $OC_2H_5$ | $CH_3$ | $OCH_3$ | $OCH_3$ | CH | |
| 422 | $CH_3CH=C$ | $OC_2H_5$ | H | $OCH_3$ | Cl | CH | |
| 423 | $CH_3CH=C$ | $OC_2H_5$ | H | $OCH_3$ | $NHCH_3$ | CH | |
| 424 | $CH_3CH=C$ | $OC_2H_5$ | H | $OCH_3$ | Br | CH | |
| 425 | $CH_3CH=C$ | $OC_2H_5$ | H | $OCH_3$ | $SCH_3$ | CH | |
| 426 | $CH_3CH=C$ | $OC_2H_5$ | H | $OCHF_2$ | $OCH_3$ | CH | |
| 427 | $CH_3CH=C$ | $OC_2H_5$ | H | $OCH_3$ | $CH_3$ | N | |
| 428 | $CH_3CH=C$ | $OC_2H_5$ | H | $OC_2H_5$ | $NHCH_3$ | N | |
| 429 | $CH_3CH=C$ | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 430 | $C_2H_5CH=C$ | $OC_2H_5$ | H | $CH_3$ | $CH_3$ | CH | |
| 431 | $C_2H_5CH=C$ | $OC_2H_5$ | H | $OCH_3$ | $CH_3$ | CH | |
| 432 | $C_2H_5CH=C$ | $OC_2H_5$ | H | $OCH_3$ | $OCH_3$ | CH | |
| 433 | $C_2H_5CH=C$ | $OC_2H_5$ | $CH_3$ | $OCH_3$ | $OCH_3$ | CH | |
| 434 | $C_2H_5CH=C$ | $OC_2H_5$ | H | $OCH_3$ | Cl | CH | |
| 435 | $C_2H_5CH=C$ | $OC_2H_5$ | H | $OCH_3$ | $NHCH_3$ | CH | |
| 436 | $C_2H_5CH=C$ | $OC_2H_5$ | H | $OCH_3$ | Br | CH | |
| 437 | $C_2H_5CH=C$ | $OC_2H_5$ | H | $OCH_3$ | $SCH_3$ | CH | |
| 438 | $C_2H_5CH=C$ | $OC_2H_5$ | H | $OCHF_2$ | $OCH_3$ | CH | |
| 439 | $C_2H_5CH=C$ | $OC_2H_5$ | H | $OCH_3$ | $CH_3$ | N | |
| 440 | $C_2H_5CH=C$ | $OC_2H_5$ | H | $OC_2H_5$ | $NHCH_3$ | N | |
| 441 | $C_2H_5CH=C$ | $OC_2H_5$ | H | $OCH_3$ | $N(CH_3)_2$ | N | |
| 442 | 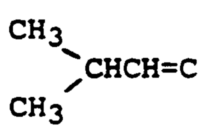 | $OC_2H_5$ | H | $CH_3$ | $CH_3$ | CH | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 443 | 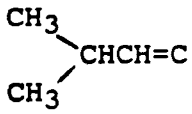 | $OC_2H_5$ | H | $OCH_3$ | $CH_3$ | CH | |
| 444 | 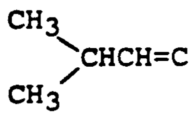 | $OC_2H_5$ | H | $OCH_3$ | $OCH_3$ | CH | Harz |
| 445 | 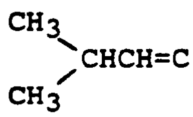 | $OC_2H_5$ | $CH_3$ | $OCH_3$ | $OCH_3$ | CH | |
| 446 | 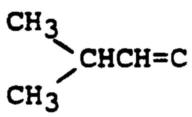 | $OC_2H_5$ | H | $OCH_3$ | Cl | CH | |
| 447 | 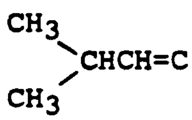 | $OC_2H_5$ | H | $OCH_3$ | $NHCH_3$ | CH | |
| 448 | 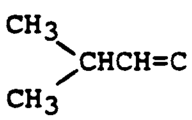 | $OC_2H_5$ | H | $OCH_3$ | Br | CH | |
| 449 | 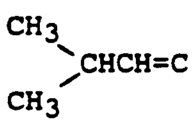 | $OC_2H_5$ | H | $OCH_3$ | $SCH_3$ | CH | |
| 450 | 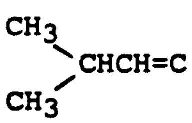 | $OC_2H_5$ | H | $OCHF_2$ | $OCH_3$ | CH | |
| 451 | 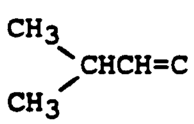 | $OC_2H_5$ | H | $OCH_3$ | $CH_3$ | N | |
| 452 | 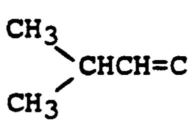 | $OC_2H_5$ | H | $OC_2H_5$ | $NHCH_3$ | N | |
| 453 | 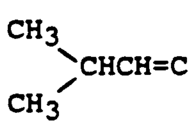 | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 454 | 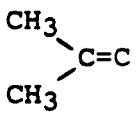 | $OC_2H_5$ | H | $CH_3$ | $CH_3$ | CH | |
| 455 | 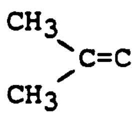 | $OC_2H_5$ | H | $OCH_3$ | $CH_3$ | CH | |
| 456 | 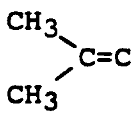 | $OC_2H_5$ | H | $OCH_3$ | $OCH_3$ | CH | |
| 457 | 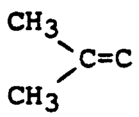 | $OC_2H_5$ | $CH_3$ | $OCH_3$ | $OCH_3$ | CH | |
| 458 | 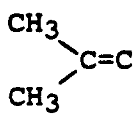 | $OC_2H_5$ | H | $OCH_3$ | Cl | CH | |
| 459 | 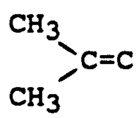 | $OC_2H_5$ | H | $OCH_3$ | $NHCH_3$ | CH | |
| 460 | 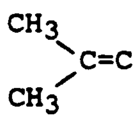 | $OC_2H_5$ | H | $OCH_3$ | Br | CH | |
| 461 | 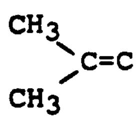 | $OC_2H_5$ | H | $OCH_3$ | $SCH_3$ | CH | |
| 462 | 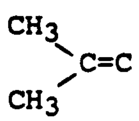 | $OC_2H_5$ | H | $OCHF_2$ | $OCH_3$ | CH | |
| 463 | 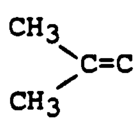 | $OC_2H_5$ | H | $OCH_3$ | $CH_3$ | N | |
| 464 | 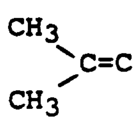 | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 465 | 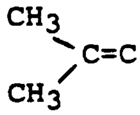 | $OC_2H_5$ | H | $OCH_3$ | $N(CH_3)_2$ | N | |
| 466 | 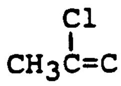 | $OC_2H_5$ | H | $CH_3$ | $CH_3$ | CH | |
| 467 | 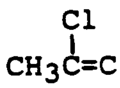 | $OC_2H_5$ | H | $OCH_3$ | $CH_3$ | CH | |
| 468 | 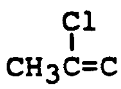 | $OC_2H_5$ | H | $OCH_3$ | $OCH_3$ | CH | |
| 469 | 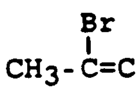 | $OC_2H_5$ | $CH_3$ | $OCH_3$ | $OCH_3$ | CH | |
| 470 | 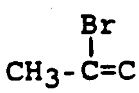 | $OC_2H_5$ | H | $OCH_3$ | Cl | CH | |
| 471 | 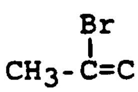 | $OC_2H_5$ | H | $OCH_3$ | $NHCH_3$ | CH | |
| 472 | $CF_3$-CH=C | $OC_2H_5$ | H | $OCH_3$ | Br | CH | |
| 473 | $CCl_3$-CH=C | $OC_2H_5$ | H | $OCH_3$ | $SCH_3$ | CH | |
| 474 | $CCl_3$-CH=C | $OC_2H_5$ | H | $OCHF_2$ | $OCH_3$ | CH | |
| 475 | $CCl_3$-CH=C | $OC_2H_5$ | H | $OCH_3$ | $CH_3$ | N | |
| 476 | 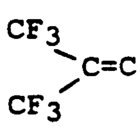 | $OC_2H_5$ | H | $OC_2H_5$ | $NHCH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 477 | 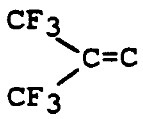 | $OC_2H_5$ | H | $OCH_3$ | $N(CH_3)_2$ | N | |
| 478 | $C_6H_5-CH=C$ | $OC_2H_5$ | H | $CH_3$ | $CH_3$ | CH | |
| 479 | $C_6H_5-CH=C$ | $OC_2H_5$ | H | $OCH_3$ | $CH_3$ | CH | |
| 480 | $C_6H_5-CH=C$ | $OC_2H_5$ | H | $OCH_3$ | $OCH_3$ | CH | |
| 481 | $C_6H_5-CH=C$ | $OC_2H_5$ | $CH_3$ | $OCH_3$ | $OCH_3$ | CH | |
| 482 | $C_6H_5-CH=C$ | $OC_2H_5$ | H | $OCH_3$ | Cl | CH | |
| 483 | $C_6H_5-CH=C$ | $OC_2H_5$ | H | $OCH_3$ | $NHCH_3$ | CH | |
| 484 | $C_6H_5-CH=C$ | $OC_2H_5$ | H | $OCH_3$ | Br | CH | |
| 485 | $C_6H_5-CH=C$ | $OC_2H_5$ | H | $OCH_3$ | $SCH_3$ | CH | |
| 486 | $C_6H_5-CH=C$ | $OC_2H_5$ | H | $OCHF_2$ | $OCH_3$ | CH | |
| 487 | $C_6H_5-CH=C$ | $OC_2H_5$ | H | $OCH_3$ | $CH_3$ | N | |
| 488 | $C_6H_5-CH=C$ | $OC_2H_5$ | H | $OC_2H_5$ | $NHCH_3$ | N | |
| 489 | $C_6H_5-CH=C$ | $OC_2H_5$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 490 | $CH_3$-CH | $N(CH_3)_2$ | H | $CH_3$ | $CH_3$ | CH | |
| 491 | $CH_3$-CH | $N(CH_3)_2$ | H | $OCH_3$ | $CH_3$ | CH | |
| 492 | $CH_3$-CH | $N(CH_3)_2$ | H | $OCH_3$ | $OCH_3$ | CH | |
| 493 | $CH_3$-CH | $N(CH_3)_2$ | $CH_3$ | $OCH_3$ | $OCH_3$ | CH | |
| 494 | $CH_3$-CH | $N(CH_3)_2$ | H | $OCH_3$ | Cl | CH | |
| 495 | $CH_3$-CH | $N(CH_3)_2$ | H | $OCH_3$ | $NHCH_3$ | CH | |
| 496 | $CH_3$-CH | $N(CH_3)_2$ | H | $OCH_3$ | Br | CH | |
| 497 | $CH_3$-CH | $N(CH_3)_2$ | H | $OCH_3$ | $SCH_3$ | CH | |
| 498 | $CH_3$-CH | $N(CH_3)_2$ | H | $OCHF_2$ | $OCH_3$ | CH | |
| 499 | $CH_3$-CH | $N(CH_3)_2$ | H | $OCH_3$ | $CH_3$ | N | |
| 500 | $CH_3$-CH | $N(CH_3)_2$ | H | $OC_2H_5$ | $NHCH_3$ | N | |
| 501 | $CH_3$-CH | $N(CH_3)_2$ | H | $OCH_3$ | $N(CH_3)_2$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 502 | $C_2H_5$-CH | $N(CH_3)_2$ | $CH_3$ | $OCH_3$ | $OCH_3$ | CH | |
| 503 | $C_2H_5$-CH | $N(CH_3)_2$ | H | $OCH_3$ | $OCH_3$ | CH | 184-186 |
| 504 | $C_2H_5$-CH | $N(CH_3)_2$ | H | $OCH_3$ | Cl | CH | |
| 505 | $C_2H_5$-CH | $N(CH_3)_2$ | H | $OCH_3$ | $CH_3$ | N | |
| 506 | $C_3H_7$-CH | $N(CH_3)_2$ | H | $OCH_3$ | $OCH_3$ | CH | |
| 507 | $C_3H_7$-CH | $N(CH_3)_2$ | H | $OCH_3$ | $CH_3$ | N | |
| 508 | $C_3H_7$-CH | $N(CH_3)_2$ | H | $OCH_3$ | Cl | CH | |
| 509 | $C_3H_7$-CH | $N(CH_3)_2$ | H | $OCH_3$ | $OCH_3$ | CH | |
| 510 | $C_3H_7$-CH | $N(CH_3)_2$ | H | $OCH_3$ | $CH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 511 | $CH_3CH{=}C$ | $N(CH_3)_2$ | $CH_3$ | $OCH_3$ | $OCH_3$ | CH | |
| 512 | $CH_3CH{=}C$ | $N(CH_3)_2$ | H | $OCH_3$ | $OCH_3$ | CH | |
| 513 | $CH_3CH{=}C$ | $N(CH_3)_2$ | H | $OCH_3$ | Cl | CH | |
| 514 | $CH_3CH_2CH{=}C$ | $N(CH_3)_2$ | H | $OCH_3$ | $CH_3$ | N | |
| 515 | 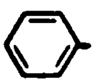 CH=C | $N(CH_3)_2$ | H | $OCH_3$ | $OCH_3$ | CH | 142 |
| 516 | " | $N(CH_3)_2$ | H | $OCH_3$ | $CH_3$ | N | |
| 517 | " | $N(CH_3)_2$ | H | $OCH_3$ | Cl | CH | |
| 518 | " | $N(CH_3)_2$ | H | $OCH_3$ | $OCH_3$ | CH | |
| 519 | " | $N(CH_3)OCH_3$ | H | $OCH_3$ | $CH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 520 | 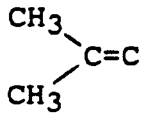 | $N(CH_3)_2$ | $CH_3$ | $OCH_3$ | $OCH_3$ | CH | |
| 521 | 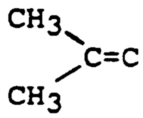 | $N(CH_3)_2$ | H | $OCH_3$ | $OCH_3$ | CH | |
| 522 | 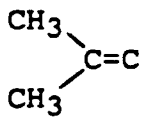 | $N(CH_3)_2$ | H | $OCH_3$ | Cl | CH | |
| 523 | 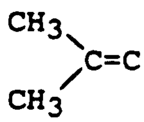 | $N(CH_3)_2$ | H | $OCH_3$ | $CH_3$ | N | |
| 524 | 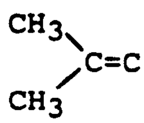 | $N(CH_3)_2$ | H | $OCH_3$ | $OCH_3$ | CH | |
| 525 | 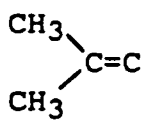 | $N(CH_3)_2$ | H | $OCH_3$ | $CH_3$ | N | |
| 526 | 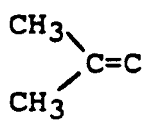 | $N(CH_3)_2$ | H | $OCH_3$ | Cl | CH | |
| 527 | 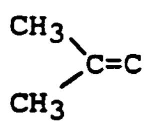 | $N(CH_3)_2$ | H | $OCH_3$ | $OCH_3$ | CH | |
| 528 | 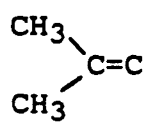 | $N(CH_3)_2$ | H | $OCH_3$ | $CH_3$ | N | |

**Fortsetzung der Tabelle 1**

| Bsp. Nr. | A | $XR^1$ | $R^2$ | $R^6$ | $R^{6'}$ | E | Fp |
|---|---|---|---|---|---|---|---|
| 529 | $C_2H_5$-CH | $NHCH_3$ | $CH_3$ | $OCH_3$ | $OCH_3$ | CH | |
| 530 | $C_2H_5$-CH | $SC_2H_5$ | H | $OCH_3$ | $OCH_3$ | CH | |
| 531 | $(CH_3)_2C$ | $OC_2H_5$ | H | $OCH_3$ | Cl | CH | |
| 532 | $C_2H_5(CH_3)C$ | $OC_2H_5$ | H | $OCH_3$ | $CH_3$ | N | |
| 533 | $C_6H_5$-$CH_2(CH_3)C$ | $OC_2H_5$ | H | $OCH_3$ | $OCH_3$ | CH | |
| 536 | $CH_2$=$CHCH_2(CH_2)C$ | $OC_2H_5$ | H | $OCH_3$ | $OCH_3$ | CH | |
| 537 | $CH_2$=$CHCH_2(CH_2)C$ | $OC_2H_5$ | H | $OCH_3$ | $CH_3$ | N | |

**Tabelle 2** (Y = S, $R^3$ = 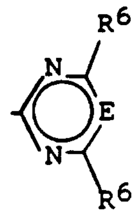 )

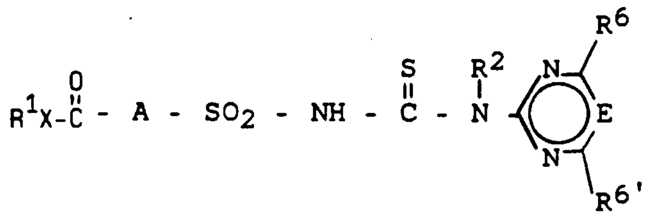

| Bsp. Nr. | A | $XR^1$ | $R^2$ | $R^6$ | $R^{6'}$ | E | Fp °C |
|---|---|---|---|---|---|---|---|
| 538 | $C_2H_5$-CH | $OCH_3$ | H | $OCH_3$ | $OCH_3$ | CH | |
| 539 | $C_2H_5$-CH | $OCH_3$ | $CH_3$ | $OCH_3$ | $OCH_3$ | CH | |
| 540 | $C_2H_5$-CH | $OC_2H_5$ | H | $OCH_3$ | $OCH_3$ | CH | |
| 541 | $C_6H_5$-CH=C | $OC_2H_5$ | H | $OCH_3$ | $CH_3$ | N | |
| 542 | $C_2H_5$-CH | $N(CH_3)_2$ | H | $OCH_3$ | $OCH_3$ | CH | |
| 543 | $C_6H_5$-$CH_2$CH | $OCH_3$ | H | $OCH_3$ | $CH_3$ | N | |
| 544 | $CH_2$=$CHCH_2$-CH | $OCH_3$ | H | $OCH_3$ | $OCH_3$ | CH | |
| 545 | CH≡C-$CH_2$CH | $OCH_3$ | H | $OCH_3$ | Cl | CH | |
| 546 | $CCl_3$CH=C | $OCH_3$ | H | $OCH_3$ | $OCH_3$ | CH | |

**Tabelle 3** (Y = O, $R^2$ = H)

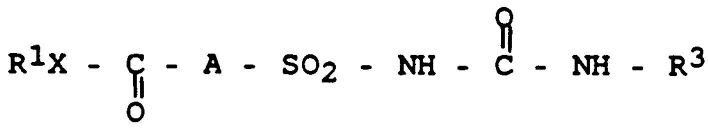

| Bsp. Nr. | A | $XR^1$ | $R^3$ | Fp °C |
|---|---|---|---|---|
| 547 | $CH_2$ | $OCH_3$ | 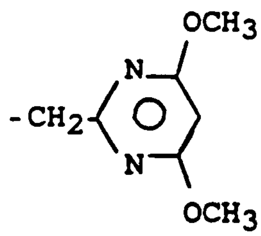 | |
| 548 | $CH_2$ | $OC_2H_5$ | " | |
| 549 | $CH_3$-CH | $OCH_3$ | " | |
| 550 | $C_2H_5$-CH | $OC_2H_5$ | " | |
| 551 | $C_3H_7$-CH | $OCH_3$ | " | |
| 552 | $C_3H_7$-CH | $OC_2H_5$ | " | |
| 553 | $CH_2$ | $OCH_3$ | 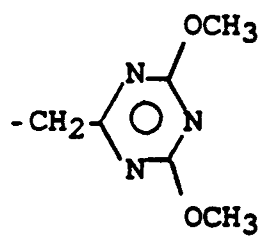 | |
| 554 | $CH_2$ | $OC_2H_5$ | " | |
| 555 | $CH_3$-CH | $OC_2H_5$ | " | |
| 556 | $C_2H_5$-CH | $OC_2H_5$ | " | |

**Fortsetzung von Tabelle 3**

| Bsp. Nr. | A | $XR^1$ | $R^3$ | Fp °C |
|---|---|---|---|---|
| 557 | $CH_2$ | $OCH_3$ | 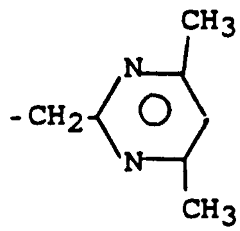 | |
| 558 | $CH_2$ | $OC_2H_5$ | " | |
| 559 | $CH_3-CH$ | $OC_2H_5$ | " | |
| 560 | $C_2H_5-CH$ | $OC_2H_5$ | " | |
| 561 | $C_3H_7-CH$ | $OC_2H_5$ | " | |
| 562 | $CH_2$ | $OCH_3$ | 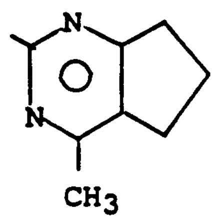 | |
| 563 | $CH_3-CH$ | $OCH_3$ | " | |
| 564 | $CH_3-CH$ | $OC_2H_5$ | " | |
| 565 | $C_2H_5-CH$ | $OC_2H_5$ | " | |
| 566 | $C_3H_7-CH$ | $OC_2H_5$ | " | |
| 567 | $CH_2$ | $OC_2H_5$ | 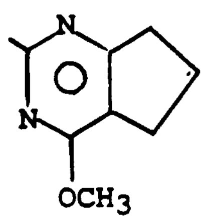 | |
| 568 | $CH_3-CH$ | $OC_2H_5$ | " | |
| 569 | $C_2H_5-CH$ | $OC_2H_5$ | " | |
| 570 | $C_3H_7-CH$ | $OC_2H_5$ | " | |

**Fortsetzung von Tabelle 3**

| Bsp. Nr. | A | $XR^1$ | $R^3$ | Fp °C |
|---|---|---|---|---|
| 571 | $CH_2$ | $OC_2H_5$ | 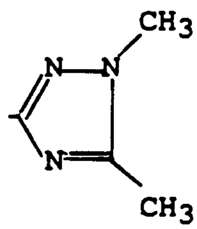 | |
| 572 | $CH_3$-CH | $OC_2H_5$ | " | |
| 573 | $C_2H_5$-CH | $OC_2H_5$ | " | |
| 574 | $C_3H_7$-CH | $OC_2H_5$ | " | |
| 575 | $CH_2$ | $OC_2H_5$ | 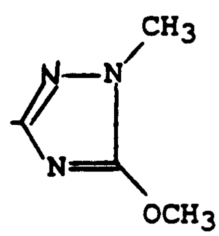 | |
| 576 | $CH_3$-CH | $OC_2H_5$ | " | |
| 577 | $C_2H_5$-CH | $OC_2H_5$ | " | |
| 578 | $C_3H_7$-CH | $OC_2H_5$ | " | |
| 579 | $CH_2$ | $OC_2H_5$ | 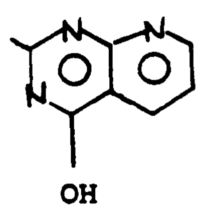 | |
| 580 | $CH_3$-CH | $OC_2H_5$ | " | |
| 581 | $C_2H_5$-CH | $OC_2H_5$ | " | |
| 582 | $C_3H_7$-CH | $OC_2H_5$ | " | |

**Fortsetzung von Tabelle 3**

| Bsp. Nr. | A | $XR^1$ | $R^3$ | Fp °C |
|---|---|---|---|---|
| 583 | $CH_2$ | $OC_2H_5$ | 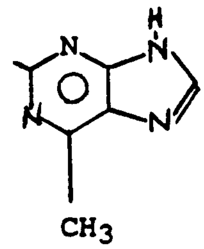 | |
| 584 | $CH_3$-CH | $OC_2H_5$ | " | |
| 585 | $C_2H_3$-CH | $OC_2H_5$ | " | |
| 586 | $C_3H_7$-CH | $OC_2H_5$ | " | |
| 587 | $CH_2$ | $OC_2H_5$ | 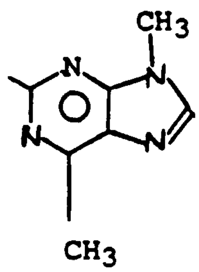 | |
| 588 | $CH_3$-CH | $OC_2H_5$ | " | |
| 589 | $C_2H_5$-CH | $OC_2H_5$ | " | |
| 590 | $C_3H_7$-CH | $OC_2H_5$ | " | |
| 591 | $CH_2$ | $OC_2H_5$ | 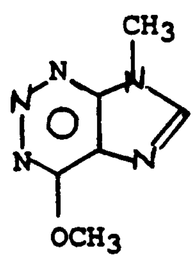 | |
| 592 | $CH_3$-CH | $OC_2H_5$ | " | |
| 593 | $C_2H_5$-CH | $OC_2H_5$ | " | |
| 594 | $C_3H_7$-CH | $OC_2H_5$ | " | |

## Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet :

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle I zeigen, weisen die erfindungsgemäßen Verbindungen eine gute herbizide Vorauflaufwirksamkeit gegen ein breites Spektrum von Ungräsern und Unkräutern auf.

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle II).

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

**Tabelle I: Vorauflaufwirkung der erfindungsgemäßen Verbindungen**

| Produkt Nr. | Dosis kg a.i./ha | herbizide Wirkung SIA | CRS | STM | LOM |
|---|---|---|---|---|---|
| 1 | 0,6 | 5 | 5 | 5 | 5 |
| 2 | 0,6 | 5 | 5 | 5 | 5 |
| 3 | 0,6 | 5 | 5 | 2 | 5 |
| 4 | 0,6 | 5 | 5 | 5 | 5 |
| 6 | 0,6 | 5 | 5 | 5 | 5 |
| 16 | 0,6 | 5 | 3 | 5 | 4 |
| 28 | 0,6 | 5 | 5 | 5 | 5 |
| 32 | 0,6 | 5 | 5 | 5 | 5 |
| 40 | 0,6 | 5 | 5 | 5 | 5 |
| 46 | 0,6 | 5 | 5 | 5 | 4 |
| 49 | 0,6 | 5 | 2 | 5 | 5 |
| 64 | 0,6 | 5 | 5 | 4 | 2 |
| 77 | 0,6 | 5 | 5 | 4 | 3 |
| 82 | 0,6 | 5 | 5 | 5 | 5 |
| 136 | 0,6 | 5 | 5 | 5 | 5 |
| 154 | 0,6 | 5 | 4 | 5 | 5 |
| 157 | 0,6 | 5 | 5 | 4 | 4 |
| 230 | 0,6 | 4 | 5 | 5 | 2 |
| 264 | 0,6 | 5 | 5 | 5 | 3 |

**Fortsetzung der Tabelle I:**

| Produkt Nr. | Dosis kg a.i./ha | herbizide Wirkung SIA | CRS | STM | LOM |
|---|---|---|---|---|---|
| 276 | 0,6 | 5 | 4 | 3 | 5 |
| 300 | 0,6 | 5 | 5 | 5 | 4 |
| 336 | 0,6 | 5 | 5 | 4 | 2 |
| 384 | 0,6 | 4 | 5 | 4 | 5 |
| 420 | 0,6 | 5 | 5 | 5 | 5 |
| 444 | 0,6 | 5 | 3 | 5 | 4 |
| 503 | 0,6 | 5 | 5 | 5 | 2 |

**Tabelle II:** Nachauflaufwirkung

| Produkt Nr. | Dosis kg a.i./ha | herbizide Wirkung SIA | CRS | STM | LOM |
|---|---|---|---|---|---|
| 1 | 0,6 | 5 | 5 | 5 | 5 |
| 2 | 0,6 | 5 | 5 | 5 | 5 |
| 3 | 0,6 | 5 | 5 | 4 | 5 |
| 4 | 0,6 | 5 | 5 | 5 | 5 |
| 6 | 0,6 | 4 | 5 | 5 | 5 |
| 16 | 0,6 | 5 | 5 | 5 | 5 |
| 28 | 0,6 | 5 | 5 | 5 | 5 |
| 32 | 0,6 | 5 | 5 | 5 | 5 |
| 40 | 0,6 | 5 | 5 | 5 | 5 |
| 46 | 0,6 | 5 | 5 | 5 | 5 |
| 49 | 0,6 | 5 | 5 | 5 | 5 |
| 65 | 0,6 | 5 | 5 | 5 | 5 |
| 77 | 0,6 | 5 | 5 | 5 | 5 |
| 82 | 0,6 | 5 | 5 | 5 | 5 |
| 136 | 0,6 | 5 | 5 | 5 | 5 |
| 154 | 0,6 | 5 | 5 | 5 | 4 |
| 157 | 0,6 | 5 | 5 | 5 | 5 |
| 230 | 0,6 | 4 | 5 | 5 | 5 |
| 264 | 0,6 | 5 | 5 | 5 | 5 |

**Fortsetzung der Tabelle II:**

| Produkt Nr. | Dosis kg a.i./ha | herbizide Wirkung SIA | CRS | STM | LOM |
|---|---|---|---|---|---|
| 276 | 0,6 | 5 | 4 | 5 | 5 |
| 300 | 0,6 | 4 | 5 | 5 | 5 |
| 336 | 0,6 | 5 | 5 | 5 | 4 |
| 384 | 0,6 | 5 | 5 | 5 | 5 |
| 420 | 0,6 | 5 | 5 | 5 | 5 |
| 444 | 0,6 | 5 | 5 | 5 | 5 |
| 503 | 0,6 | 5 | 5 | 5 | 5 |

**Abkürzungen:**

**SIA = Sinapis alba**

**CRS = Chrysanthemum segetum**

**STM = Stellaria media**

**LOM = Lolium multiflorum**

Wuchshemmung an Getreide

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 % den Stillstand des Wachstums und 0 % ein Wachstum entsprechend den unbehandelten Kontrollpflanzen bedeuten. Es zeigte sich, daß die Verbindungen sehr gute wachstumsregulierende Eigenschaften besitzen. Die Ergebnisse sind in der nachfolgenden Tabelle III zusammengestellt.

Tabelle III

| Verbindungen nach Bsp.-Nr. | Anwendungskonz. kg/ha | Wuchshemmung (%) | | | Phytotox. Wirkung |
|---|---|---|---|---|---|
| | | Weizen | Gerste | Roggen | |
| 1 | 0,62<br>0,31 | 22<br>17 | 32<br>24 | 19<br>17 | keine<br>Schäden |
| 16 | 0,62<br>0,31 | 23<br>17 | 37<br>22 | 24<br>16 | keine<br>Schäden |
| 154 | 0,62<br>0,31 | 21<br>16 | 34<br>21 | 24<br>17 | keine<br>Schäden |
| 276 | 0,62<br>0,31 | 24<br>19 | 26<br>23 | 23<br>18 | keine<br>Schäden |

**Patentansprüche**
**Patentansprüche für folgende Vertragsstaaten : AT, BE, CH, DE, FR, GB, IT, LI, NL, SE**

1. Verbindungen der Formel I

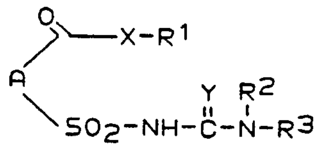

(I) ,

worin

A = einen Rest der Formel $>C(R^4)_2$ oder $>C=C(R^5)_2$,

$R^1$ = H, $(C_1$-$C_8)$Alkyl, $(C_2$-$C_8)$Alkenyl, $(C_2$-$C_8)$Alkinyl, wobei diese aliphatischen Reste ein- oder mehrfach durch Halogen, oder ein- oder zweifach durch $(C_1$-$C_6)$Alkoxy, $(C_2$-$C_6)$-Alkenyloxy, $(C_2$-$C_6)$Alkinyloxy, $(C_1$-$C_6)$Alkylthio, $(C_1$-$C_6)$Alkylsulfinyl, $(C_1$-$C_6)$Alkylsulfonyl, $(C_1$-$C_6)$Alkoxycarbonyl oder durch Phenyl Substituiert sein können; $(C_3$-$C_8)$Cycloalkyl, das ein- oder mehrfach durch Halogen, oder ein- oder zweifach durch $(C_1$-$C_4)$Alkoxy oder $(C_1$-$C_4)$-Alkylthio substituiert sein kann; $(C_5$-$C_8)$Cycloalkenyl, Cyclopropylmethyl, Epoxypropyl, Furfuryl, Tetrahydrofurfuryl, Phenoxy$(C_1$-$C_6)$alkyl oder Phenyl, die beide im Phenyl-Ring durch Halogen, $(C_1$-$C_4)$Alkyl, $(C_1$-$C_4)$Alkoxy oder $NO_2$ substituiert sein können,

$R^2$ = H, $(C_1$-$C_8)$Alkyl, $(C_2$-$C_8)$Alkenyl, $(C_2$-$C_8)$Alkinyl oder $(C_1$-$C_4)$Alkoxy,

$R^3$ = einen Rest der Formel

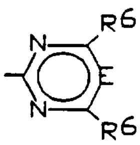 , 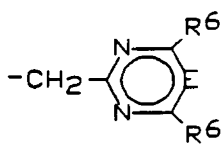 , 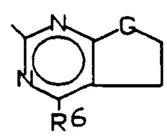 ,

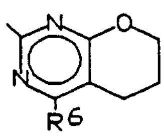 , 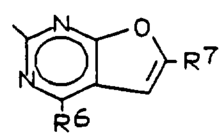 , 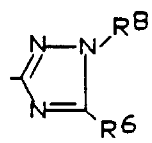 ,

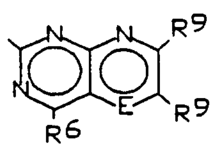 oder 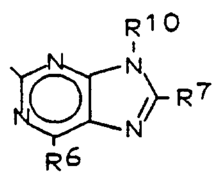

$R^4$ = unabhängig voneinander H, $(C_1$-$C_6)$Alkyl, $(C_2$-$C_6)$Alkenyl, $(C_2$-$C_6)$Alkinyl, $(C_3$-$C_8)$-Cycloalkyl, wobei die vorgenannten C-haltigen Reste ein- oder mehrfach durch Halogen oder ein- oder zweifach durch $(C_1$-$C_6)$Alkoxy, $(C_2$-$C_6)$Alkenyloxy, $(C_2$-$C_6)$Alkinyloxy, $(C_1$-$C_6)$Alkylthio, $(C_1$-$C_6)$Alkylsulfinyl, $(C_1$-$C_6)$Alkylsulfonyl oder $(C_1$-$C_6)$Alkoxycarbonyl oder durch Phenyl, das ein- oder mehrfach durch Halogen, $(C_1$-$C_4)$Alkyl, $(C_1$-$C_4)$Alkoxy, $(C_1$-$C_4)$Alkylthio, $(C_1$-$C_4)$Alkoxycarbonyl oder $NO_2$ substituiert sein kann, substituiert sein können, ferner einer der beiden Reste $R^4$ Phenyl, das ein- oder mehrfach durch Halogen, $(C_1$-$C_4)$Alkyl, $(C_1$-$C_4)$Alkoxy, $NO_2$ oder $CF_3$ substituiert sein kann,

$R^5$ = unabhängig voneinander H, $(C_1$-$C_6)$Alkyl, das ein- oder mehrfach durch Halogen oder ein- oder zweifach durch $(C_1$-$C_6)$Alkoxy, $(C_1$-$C_6)$Alkylthio, $(C_1$-$C_6)$Alkylsulfinyl, oder $(C_1$-$C_6)$-Alkoxycarbonyl substituiert sein kann oder einer der Reste $R^5$ Phenyl, das ein- oder mehrfach durch Halogen, $(C_1$-$C_4)$Alkyl, $(C_1$-$C_4)$Alkoxy, $NO_2$, $CF_3$ oder $(C_1$-$C_6)$-Alkoxycarbonyl substituiert sein kann, oder beide Reste $R^5$ gemeinsam einen Alkylenrest $(CH_2)_n$,

$R^6$ = unabhängig voneinander H, Halogen, $(C_1\text{-}C_6)$Alkyl, $(C_1\text{-}C_6)$Alkoxy oder $(C_1\text{-}C_6)$Alkylthio, die ein- oder mehrfach durch Halogen oder ein- oder zweifach durch $(C_1\text{-}C_4)$Alkoxy oder $(C_1\text{-}C_4)$Alkylthio substituiert sein können; einen Rest $N(R^{11})_2$, $(C_3\text{-}C_6)$Cycloalkyl, $-OCHR^7COOR^{11}$, $(C_3\text{-}C_5)$Alkenyl, $(C_2\text{-}C_4)$Alkinyl, $(C_3\text{-}C_5)$Alkenyloxy oder $(C_3\text{-}C_5)$-Alkinyloxy,

$R^7$ = H oder $(C_1\text{-}C_4)$Alkyl,

$R^8$ = $(C_1\text{-}C_4)$Alkyl, $-CHF_2$ oder $-CH_2CF_3$,

$R^9$ = unabhängig voneinander H, $(C_1\text{-}C_4)$Alkyl, $(C_1\text{-}C_4)$Alkoxy oder Halogen,

$R^{10}$ = H, $(C_1\text{-}C_4)$Alkyl, $-CHF_2$ oder $-CH_2CF_3$,

$R^{11}$ = unabhängig voneinander H, $(C_1\text{-}C_4)$Alkyl, $(C_2\text{-}C_4)$Alkenyl oder $(C_3\text{-}C_4)$Alkinyl,

E = CH oder N,

G = $CH_2$ oder O,

X = O, S oder $NR^{12}$,

Y = O oder S,

$R^{12}$ = H, $(C_1\text{-}C_6)$Alkyl, $(C_2\text{-}C_6)$Alkenyl, $(C_2\text{-}C_6)$Alkinyl, $(C_1\text{-}C_6)$Alkoxy, oder einen Rest der Formel

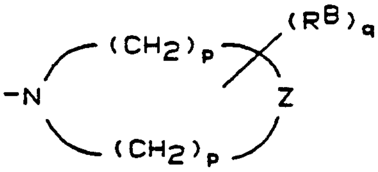

n = eine ganze Zahl von 3 bis 6,

p = eine ganze Zahl von 1 bis 3,

q = eine ganze Zahl von 0 bis 3 und

Z = O, S, $CH_2$, NH oder $N(C_1\text{-}C_4\text{-Alkyl})$ bedeuten, oder deren für die Landwirtschaft einsetzbaren Salze.

**2.** Verbindungen der Formel I nach Anspruch 1, dadurch gekennzeichnet, daß

A einen Rest $>C(R^4)_2$ oder $>C = C(R^5)_2$

$R^1$ $(C_1\text{-}C_4)$Alkyl, das ein- oder mehrfach durch Halogen oder ein- bis zweifach durch $(C_1\text{-}C_4)$-Alkoxy substituiert sein kann,

$R^2$ H, $(C_1\text{-}C_4)$Alkyl oder Allyl,

$R^3$ einen Rest der Formel

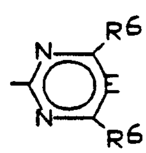 ,

$R^4$ unabhängig voneinander H oder $(C_1\text{-}C_6)$Alkyl, das gegebenenfalls ein- oder mehrfach durch Halogen oder ein- bis zweifach durch $(C_1\text{-}C_4\text{-})$Alkoxy oder $(C_1\text{-}C_4)$-Alkylthio substituiert sein kann, oder ein Rest $R^4$ $(C_2\text{-}C_6)$Alkenyl oder Phenyl, das ein- oder mehrfach durch Halogen, $(C_1\text{-}C_4)$-Alkyl, $(C_1\text{-}C_4)$-Alkoxy, $NO_2$ oder $CF_3$ substituiert sein kann, und der andere Rest $R^4$ für Wasserstoff,

$R^5$ unabhängig voneinander H oder $(C_1\text{-}C_6)$Alkyl, das gegebenenfalls ein- oder mehrfach durch Halogen substituiert sein kann,

$R^6$ unabhängig voneinander Halogen, $(C_1\text{-}C_4)$Alkyl oder $(C_1\text{-}C_4)$Alkoxy, die beide halogeniert sein können,

E CH oder N,

X O, S oder $NR^{12}$,

Y O oder S und

$R^{12}$ H, $(C_1\text{-}C_4)$Alkyl oder $(C_2\text{-}C_4)$Alkenyl bedeuten.

**3.** Verbindungen nach Anspruch 1 oder 2, dadurch gekennzeichnet, daß ein Rest $R^4$ für ($C_1$-$C_4$)-Alkyl oder für Phenyl, das ein- bis dreifach durch Fluor, Chlor, ($C_1$-$C_4$)-Alkoxy, ($C_1$-$C_4$)-Alkyl oder Nitro substituiert ist, steht und der andere Rest $R^4$ für Wasserstoff steht.

**4.** Verbindungen nach einem der Ansprüche 1 bis 3, dadurch gekennzeichnet, daß $R^1$ ($C_1$-$C_4$)-Alkyl und $R^2$ Wasserstoff bedeuten.

**5.** Verbindungen nach einem der Ansprüche 1 bis 4, dadurch gekennzeichnet, daß $R^6$ $CH_3$, $OCH_3$, $OC_2H_5$, Cl oder $OCF_2H$ bedeutet.

**6.** Verfahren zur Herstellung der Verbindungen der Formel I von Anspruch 1 oder deren Salze, dadurch gekennzeichnet, daß man

(a) eine Verbindung der Formel (II)

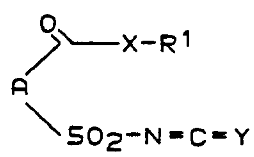

(II) ,

wobei A, X, Y, $R^1$ die in Anspruch 1 angegebene Bedeutung haben, mit der Ausnahme, daß $R^1 \neq H$ und $X \neq NH$ sind, mit einer Verbindung der Formel (III)

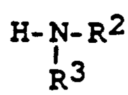

(III)

umsetzt,

(b) eine Verbindung der Formel (IV)

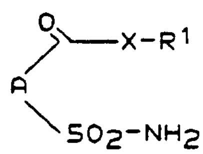

(IV)

mit einem (Thio)-Carbamat der Formel (V)

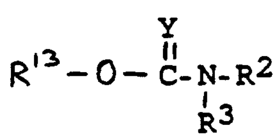

(V) ,

wobei $R^{13}$ ($C_1$-$C_6$)Alkyl, ($C_1$-$C_4$)Halogenalkyl oder Phenyl bedeutet, das ein- oder mehrfach durch Halogen, ($C_1$-$C_4$)Alkyl, oder $NO_2$ substituiert sein kann, umsetzt,

(c) ein (Thio)-Carbamat der Formel (VI) mit einer Verbindung der Formel (III)

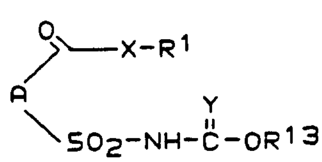

(VI),

wobei $R^{13}$ die oben angegebene Bedeutung hat, umsetzt oder
(d) eine Carbonsäure der Formel (VII)

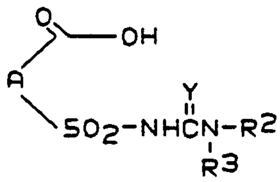

(VII)

mit einem Alkylierungsreagens der Formel (VIII)

$R^1$-X (VIII)

wobei X für eine nucleofuge Abgangsgruppe steht, umsetzt und die erhaltenen Verbindungen gegebenenfalls in ihre Salze überführt.

**7.** Herbizide oder pflanzenwachstumsregulierende Mittel, dadurch gekennzeichnet, daß sie eine wirksame Menge einer Verbindung der Formel I nach einem der Ansprüche 1 bis 5, deren Stereoisomer oder deren Salz und inerte Hilfsstoffe enthalten.

**8.** Verwendung der Verbindungen der Formel I nach einem der Ansprüche 1 bis 5, deren Stereoisomeren oder deren Salze als Herbizide oder Pflanzenwachstumsregulatoren.

**9.** Verfahren zur Bekämpfung von Schadpflanzen, dadurch gekennzeichnet, daß man auf diese oder die landwirtschaftlich oder industriell genutzten Böden eine wirksame Menge einer nach einem der Ansprüche 1 bis 5 definierten Verbindung der Formel I oder deren Stereoisomeren oder deren Salz appliziert.

**10.** Verfahren zur Wachstumsregulation von Nutzpflanzen, dadurch gekennzeichnet, daß man auf diese oder die Anbaufläche eine wirksame Menge einer Verbindung der Formel I nach einem der Ansprüche 1 bis 5 oder deren Stereoisomer oder deren Salz appliziert.

**Patentansprüche für folgenden Vertragsstaat : ES**

**1.** Verfahren zur Herstellung von Verbindungen der Formel I

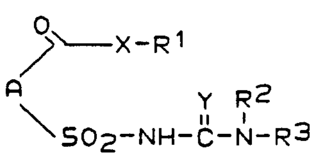

(I) ,

worin
A = einen Rest der Formel >$C(R^4)_2$ oder >$C=C(R^5)_2$,
$R^1$ = H, ($C_1$-$C_8$)Alkyl, ($C_2$-$C_8$)Alkenyl, ($C_2$-$C_8$)Alkinyl, wobei diese aliphatischen Reste ein- oder mehrfach durch Halogen, oder ein- oder zweifach durch ($C_1$-$C_6$)Alkoxy, ($C_2$-$C_6$)-Alkenyloxy, ($C_2$-$C_6$)Alkinyloxy, ($C_1$-$C_6$)Alkylthio, ($C_1$-$C_6$)Alkylsulfinyl, ($C_1$-$C_6$)Alkylsulfonyl, ($C_1$-$C_6$)Alkoxycarbonyl oder durch Phenyl substituiert sein können; ($C_3$-$C_8$)Cycloalkyl, das ein- oder mehrfach durch Halogen, oder ein- oder zweifach durch ($C_1$-$C_4$)Alkoxy oder ($C_1$-$C_4$)-Alkylthio substituiert sein kann; ($C_5$-$C_8$)Cycloalkenyl, Cyclopropylmethyl, Epoxypropyl, Furfuryl, Tetrahydrofurfuryl, Phenoxy($C_1$-$C_6$)alkyl oder Phenyl, die beide im Phenyl-Ring durch Halogen, ($C_1$-$C_4$)Alkyl, ($C_1$-$C_4$)Alkoxy oder $NO_2$ substituiert sein können,
$R^2$ = H, ($C_1$-$C_8$)Alkyl, ($C_2$-$C_8$)Alkenyl, ($C_2$-$C_8$)Alkinyl oder ($C_1$-$C_4$)Alkoxy,
$R^3$ = einen Rest der Formel

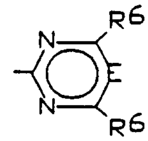 , 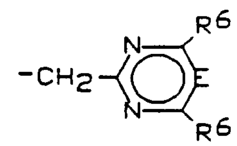 , 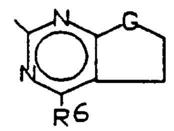 ,

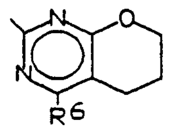 , 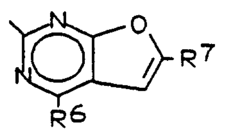 , 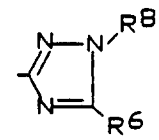 ,

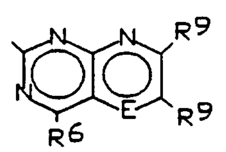 oder 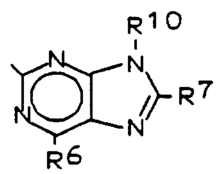

$R^4$ = unabhängig voneinander H, $(C_1\text{-}C_6)$Alkyl, $(C_2\text{-}C_6)$Alkenyl, $(C_2\text{-}C_6)$Alkinyl, $(C_3\text{-}C_8)$-Cycloalkyl, wobei die vorgenannten C-haltigen Reste ein- oder mehrfach durch Halogen oder ein- oder zweifach durch $(C_1\text{-}C_6)$Alkoxy, $(C_2\text{-}C_6)$Alkenyloxy, $(C_2\text{-}C_6)$Alkinyloxy, $(C_1\text{-}C_6)$Alkylthio, $(C_1\text{-}C_6)$Alkylsulfinyl, $(C_1\text{-}C_6)$Alkylsulfonyl oder $(C_1\text{-}C_6)$Alkoxycarbonyl oder durch Phenyl, das ein- oder mehrfach durch Halogen, $(C_1\text{-}C_4)$Alkyl, $(C_1\text{-}C_4)$Alkoxy, $(C_1\text{-}C_4)$Alkylthio, $(C_1\text{-}C_4)$Alkoxycarbonyl oder $NO_2$ substituiert sein kann, substituiert sein können, ferner einer der beiden Reste $R^4$ Phenyl, das ein- oder mehrfach durch Halogen, $(C_1\text{-}C_4)$Alkyl, $(C_1\text{-}C_4)$Alkoxy, $NO_2$ oder $CF_3$ substituiert sein kann,

$R^5$ = unabhängig voneinander H, $(C_1\text{-}C_6)$Alkyl, das ein- oder mehrfach durch Halogen oder ein- oder zweifach durch $(C_1\text{-}C_6)$Alkoxy, $(C_1\text{-}C_6)$Alkylthio, $(C_1\text{-}C_6)$Alkylsulfinyl, oder $(C_1\text{-}C_6)$-Alkoxycarbonyl substituiert sein kann oder einer der Reste $R^5$ Phenyl, das ein- oder mehrfach durch Halogen, $(C_1\text{-}C_4)$Alkyl, $(C_1\text{-}C_4)$Alkoxy, $NO_2$, $CF_3$ oder $(C_1\text{-}C_6)$-Alkoxycarbonyl substituiert sein kann, oder beide Reste $R^5$ gemeinsam einen Alkylenrest $(CH_2)_n$,

$R^6$ = unabhängig voneinander H, Halogen, $(C_1\text{-}C_6)$Alkyl, $(C_1\text{-}C_6)$Alkoxy oder $(C_1\text{-}C_6)$Alkylthio, die ein- oder mehrfach durch Halogen oder ein- oder zweifach durch $(C_1\text{-}C_4)$Alkoxy oder $(C_1\text{-}C_4)$Alkylthio substituiert sein können; einen Rest $N(R^{11})_2$, $(C_3\text{-}C_6)$Cycloalkyl, $-OCHR^7COOR^{11}$, $(C_3\text{-}C_5)$Alkenyl, $(C_2\text{-}C_4)$Alkinyl, $(C_3\text{-}C_5)$Alkenyloxy oder $(C_3\text{-}C_5)$-Alkinyloxy,

$R^7$ = H oder $(C_1\text{-}C_4)$Alkyl,

$R^8$ = $(C_1\text{-}C_4)$Alkyl, $-CHF_2$ oder $-CH_2CF_3$,

$R^9$ = unabhängig voneinander H, $(C_1\text{-}C_4)$Alkyl, $(C_1\text{-}C_4)$Alkoxy oder Halogen,

$R^{10}$ = H, $(C_1\text{-}C_4)$Alkyl, $-CHF_2$ oder $-CH_2CF_3$,

$R^{11}$ = unabhängig voneinander H, $(C_1\text{-}C_4)$Alkyl, $(C_2\text{-}C_4)$Alkenyl oder $(C_3\text{-}C_4)$Alkinyl,

E = CH oder N,

G = $CH_2$ oder O,

X = O, S oder $NR^{12}$,

Y = O oder S,

$R^{12}$ = H, $(C_1\text{-}C_6)$Alkyl, $(C_2\text{-}C_6)$Alkenyl, $(C_2\text{-}C_6)$Alkinyl, $(C_1\text{-}C_6)$Alkoxy, oder einen Rest der Formel

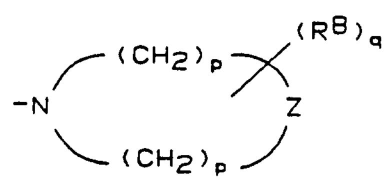

n = eine ganze Zahl von 3 bis 6,
p = eine ganze Zahl von 1 bis 3,
q = eine ganze Zahl von 0 bis 3 und
Z = O, S, $CH_2$, NH oder N($C_1$-$C_4$-Alkyl) bedeuten, oder deren für die Landwirtschaft einsetzbaren Salze,

dadurch gekennzeichnet, daß man

(a) eine Verbindung der Formel (II)

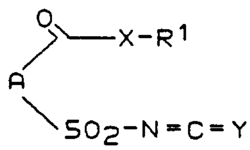

(II) ,

wobei A, X, Y, $R^1$ die in Formel (I) angegebenen Bedeutung haben, mit der Ausnahme, daß $R^1 \neq H$ und $X \neq NH$ sind, mit einer Verbindung der Formel (III)

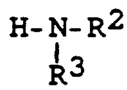

(III)

umsetzt,

(b) eine Verbindung der Formel (IV)

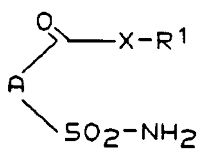

(IV)

mit einem (Thio)-Carbamat der Formel (V)

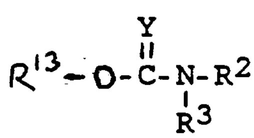

(V) ,

wobei $R^{13}$ ($C_1$-$C_6$)Alkyl, ($C_1$-$C_4$)Halogenalkyl oder Phenyl bedeutet, das ein- oder mehrfach durch Halogen, ($C_1$-$C_4$)Alkyl, oder $NO_2$ substituiert sein kann, umsetzt,

(c) ein (Thio)-Carbamat der Formel (VI) mit einer Verbindung der Formel (III)

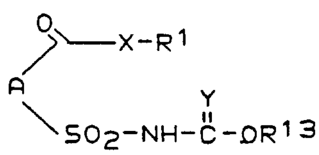

(VI),

wobei $R^{13}$ die oben angegebene Bedeutung hat, umsetzt oder

(d) eine Carbonsäure der Formel (VII)

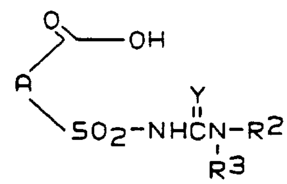

(VII)

mit einem Alkylierungsreagens der Formel (VIII)

$R^1$-X (VIII)

wobei X für eine nucleofuge Abgangsgruppe steht, umsetzt und die erhaltenen Verbindungen gegebenenfalls in ihre Salze überführt.

**2.** Verfahren nach Anspruch 1, dadurch gekennzeichnet, daß in Formel (I)

A einen Rest $>C(R^4)_2$ oder $>C = C(R^5)_2$

$R^1$ $(C_1\text{-}C_4)$Alkyl, das ein- oder mehrfach durch Halogen oder ein- bis zweifach durch $(C_1\text{-}C_4)$-Alkoxy substituiert sein kann,

$R^2$ H, $(C_1\text{-}C_4)$Alkyl oder Allyl,

$R^3$ einen Rest der Formel

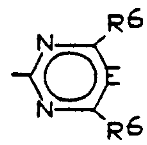

,

$R^4$ unabhängig voneinander H oder $(C_1\text{-}C_6)$Alkyl, das gegebenenfalls ein- oder mehrfach durch Halogen oder ein- bis zweifach durch $(C_1\text{-}C_4\text{-})$Alkoxy oder $(C_1\text{-}C_4)$-Alkylthio substituiert sein kann, oder ein Rest $R^4$ $(C_2\text{-}C_6)$Alkenyl oder Phenyl, das ein- oder mehrfach durch Halogen, $(C_1\text{-}C_4)$-Alkyl, $(C_1\text{-}C_4)$-Alkoxy, $NO_2$ oder $CF_3$ substituiert sein kann, und der andere Rest $R^4$ für Wasserstoff,

$R^5$ unabhängig voneinander H oder $(C_1\text{-}C_6)$Alkyl, das gegebenenfalls ein- oder mehrfach durch Halogen substituiert sein kann,

$R^6$ unabhängig voneinander Halogen, $(C_1\text{-}C_4)$Alkyl oder $(C_1\text{-}C_4)$Alkoxy, die beide halogeniert sein können,

E CH oder N,

X O, S oder $NR^{12}$,

Y O oder S und

$R^{12}$ H, $(C_1\text{-}C_4)$Alkyl oder $(C_2\text{-}C_4)$Alkenyl bedeuten.

**3.** Verfahren nach Anspruch 1 oder 2, dadurch gekennzeichnet, daß ein Rest $R^4$ für $(C_1\text{-}C_4)$-Alkyl oder für Phenyl, das ein- bis dreifach durch Fluor, Chlor, $(C_1\text{-}C_4)$-Alkoxy, $(C_1\text{-}C_4)$-Alkyl oder Nitro substituiert ist, steht und der andere Rest $R^4$ für Wasserstoff steht.

**4.** Verfahren nach einem der Ansprüche 1 bis 3, dadurch gekennzeichnet, daß $R^1$ $(C_1\text{-}C_4)$-Alkyl und $R^2$ Wasserstoff bedeuten.

**5.** Verfahren nach einem der Ansprüche 1 bis 4, dadurch gekennzeichnet, daß $R^6$ $CH_3$, $OCH_3$, $OC_2H_5$, Cl oder $OCF_2H$ bedeutet.

**6.** Verwendung der gemäß einem der Ansprüche 1 bis 5 definierten Verbindungen der Formel I, deren Stereoisomeren oder deren Salze als Herbizide oder Pflanzenwachstumsregulatoren.

7. Verfahren zur Bekämpfung von Schadpflanzen, dadurch gekennzeichnet, daß man auf diese oder die landwirtschaftlich oder industriell genutzten Böden eine wirksame Menge einer gemäß einem der Ansprüche 1 bis 5 definierten Verbindung der Formel I oder deren Stereoisomeren oder deren Salz appliziert.

8. Verfahren zur Wachstumsregulation von Nutzpflanzen, dadurch gekennzeichnet, daß man auf diese oder die Anbaufläche eine wirksame Menge einer gemäß einem der Ansprüche 1 bis 5 definierten Verbindung von Formel I oder deren Stereoisomer oder deren Salz appliziert.

## Claims

**Claims for the following Contracting States : AT, BE, CH, DE, FR, GB, IT, LI, NL, SE.**

1. A compound of the formula I

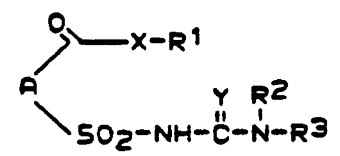

(I) ,

wherein

A denotes a radical of the formula $>C(R^4)_2$ or $>C=C(R^5)_2$,

$R^1$ denotes H, $(C_1-C_8)$alkyl, $(C_2-C_8)$alkenyl, $(C_2-C_8)$alkynyl, it being possible for these aliphatic radicals to be monosubstituted or polysubstituted by halogen, or to be monosubstitinted or disubstituted by $(C_1-C_6)$alkoxy, $(C_2-C_6)$alkenyloxy, $(C_2-C_6)$alkynyloxy, $(C_1-C_6)$alkylthio, $(C_1-C_6)$alkylsulfinyl, $(C_1-C_6)$alkylsulfonyl, $(C_1-C_6)$alkoxycarbonyl or by phenyl; $(C_3-C_8)$cycloalkyl which can be monosubstituted or polysubstituted by halogen, or monosubstituted or disubstituted by $(C_1-C_4)$alkoxy or $(C_1-C_4)$alkylthio; $(C_5-C_8)$-cycloalkenyl, cyclopropylmethyl, epoxypropyl, furfuryl, tetrahydrofurfuryl, or phenoxy$(C_1-C_6)$alkyl or phenyl, both of which can be substituted in the phenyl ring by halogen, $(C_1-C_4)$alkyl, $(C_1-C_4)$alkoxy or $NO_2$,

$R^2$ denotes H, $(C_1-C_8)$alkyl, $(C_2-C_8)$alkenyl, $(C_2-C_8)$-alkynyl or $(C_1-C_4)$alkoxy,

$R^3$ denotes a radical of the formula

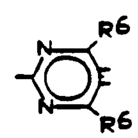 , 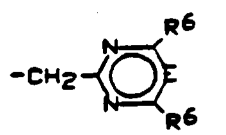 , 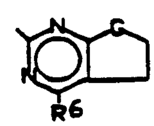 ,

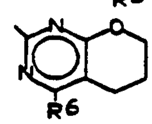 , 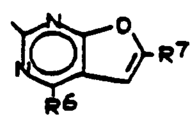 , 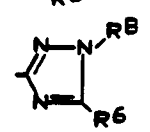 ,

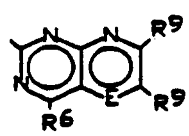 or 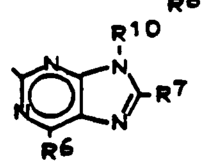

the $R^4$ radicals independently of one another denote H, $(C_1-C_6)$alkyl, $(C_2-C_6)$alkenyl, $(C_2-C_6)$-alkynyl, $(C_3-C_8)$cycloalkyl, it being possible for the abovementioned radicals which contain C to be monosubstituted or polysubstituted by halogen or monosubstituted or disubstituted by $(C_1-C_6)$alkoxy, $(C_2-C_6)$alkenyloxy, $(C_2-C_6)$alkynyloxy, $(C_1-C_6)$alkylthio, $(C_1-C_6)$alkylsulfinyl, $(C_1-C_6)$alkylsulfonyl or $(C_1-C_6)$alkoxycarbonyl, or by phenyl which can be monosubstituted or polysubstituted by halogen, $(C_1-C_4)$alkyl, $(C_1-C_4)$alkoxy, $(C_1-C_4)$alkylthio, $(C_1-C_4)$alkoxycarbonyl or $NO_2$, it being furthermore possible for one of the two radicals $R^4$ to denote phenyl which can be monosubstituted or polysubstituted by halogen, $(C_1-C_4)$alkyl, $(C_1-C_4)$alkoxy, $NO_2$ or $CF_3$,

the $R^5$ radicals independently of one another denote H, or $(C_1-C_6)$alkyl, which can be monosubstituted or polysubstituted by halogen or monosubstituted or disubstituted by $(C_1-C_6)$-alkoxy, $(C_1-C_6)$alkylthio, $(C_1-C_6)$alkylsulfinyl or $(C_1-C_6)$alkoxycarbonyl, or it being possible for one of the radicals $R^5$ to denote phenyl which can be monosubstituted or polysubstituted by halogen, $(C_1-C_4)$alkyl, $(C_1-C_4)$alkoxy, $NO_2$, $CF_3$ or $(C_1-C_6)$alkoxycarbonyl, or for the two radicals $R^5$ together to denote an alkylene radical $-(CH_2)_n-$,

the $R^6$ radicals independently of one another denote H, halogen, or $(C_1-C_6)$alkyl, $(C_1-C_6)$alkoxy or $(C_1-C_6)$-alkylthio which can be monosubstituted or polysubstituted by halogen or monosubstituted or disubstituted by $(C_1-C_4)$alkoxy or $(C_1-C_4)$alkylthio; a radical $N(R^{11})_2$, $(C_3-C_6)$cycloalkyl, $-OCHR^7COOR^{11}$, $(C_3-C_5)$alkenyl, $(C_2-C_4)$alkynyl, $(C_3-C_5)$alkenyloxy or $(C_3-C_5)$alkynyloxy,

$R^7$ denotes H or $(C_1-C_4)$alkyl,

$R^8$ denotes $(C_1-C_4)$alkyl, $-CHF_2$ or $-CH_2CF_3$,

the $R^9$ radicals independently of one another denote H, $(C_1-C_4)$alkyl, $(C_1-C_4)$alkoxy or halogen,

$R^{10}$ denotes H, $(C_1-C_4)$alkyl, $-CHF_2$ or $-CH_2CF_3$,

the $R^{11}$ radicals independently of one another denote H, $(C_1-C_4)$alkyl, $(C_2-C_4)$alkenyl or $(C_2-C_4)$-alkynyl,

E denotes CH or N,

G denotes $CH_2$ or O,

X denotes O, S or $NR^{12}$,

Y denotes O or S,

$R^{12}$ denotes H, $(C_1-C_6)$alkyl, $(C_2-C_6)$alkenyl, $(C_2-C_6)$alkynyl, $(C_1-C_6)$alkoxy, or a radical of the formula

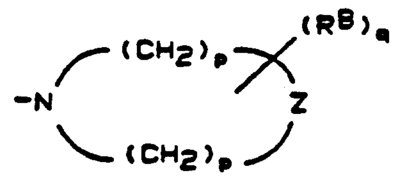

n denotes an integer from 3 to 6,

p denotes an integer from 1 to 3,

q denotes an integer from 0 to 3 and

Z denotes O, S, $CH_2$, NH or $N(C_1-C_4\text{-alkyl})$, or the salts thereof which can be employed in agriculture.

**2.** A compound of the formula I as claimed in claim 1,
wherein

A denotes a radical $>C(R^4)_2$ or $>C=C(R^5)_2$,

$R^1$ denotes $(C_1-C_4)$alkyl which can be monosubstituted or polysubstituted by halogen or monosubstituted to disubstituted by $(C_1-C_4)$alkoxy,

$R^2$ denotes H, $(C_1-C_4)$alkyl or allyl,

$R^3$ denotes a radical of the formula

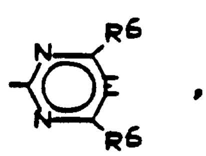

the $R^4$ radicals independently of one another denote H, or $(C_1-C_6)$alkyl which can optionally be monosubstituted or polysubstituted by halogen or monosubstituted to disubstituted by $(C_1-C_4)$alkoxy or $(C_1-C_4)$alkylthio, or a radical $R^4$ denotes $(C_2-C_6)$alkenyl or phenyl which can be monosubstituted or polysubstituted by halogen, $(C_1-C_4)$alkyl, $(C_1-C_4)$alkoxy, No2 or $CF_3$, and the other radical $R^4$ denotes hydrogen,

the $R^5$ radicals independently of one another denote H, or $(C_1-C_6)$alkyl, which can optionally be monosubstituted or polysubstituted by halogen,

| | |
|---|---|
| the $R^6$ | radicals independently of one another denote halogen, $(C_1\text{-}C_4)$alkyl or $(C_1\text{-}C_4)$alkoxy, both of which can be halogenated, |
| E | denotes CH or N, |
| X | denotes O, S or $NR^{12}$, |
| Y | denotes O or S and |
| $R^{12}$ | denotes H, $(C_1\text{-}C_4)$alkyl or $(C_2\text{-}C_4)$alkenyl. |

**3.** A compound as claimed in claim 1 or 2, wherein one radical $R^4$ is $(C_1\text{-}C_4)$alkyl or is phenyl which is monosubstituted to trisubstituted by fluorine, chlorine, $(C_1\text{-}C_4)$alkoxy, $(C_1\text{-}C_4)$-alkyl or nitro, and the other radical $R^4$ is hydrogen.

**4.** A compound as claimed in one of claims 1 to 3, wherein $R^1$ denotes $(C_1\text{-}C_4)$alkyl and $R^2$ denotes hydrogen.

**5.** A compound as claimed in one of claims 1 to 4, wherein $R^6$ denotes $CH_3$, $OCH_3$, $OC_2H_5$, Cl or $OCF_2H$.

**6.** A process for the preparation of a compound of the formula I of claim 1 or a salt thereof, which comprises reacting

(a) a compound of the formula (II)

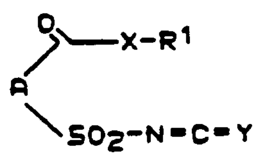

(II)

where A, X, Y, $R^1$ have the meaning indicated in claim 1, with the exception that $R^1$ is not H and X is not NH, with a compound of the formula (III)

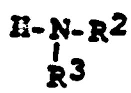

(III)

(b) a compound of the formula (IV)

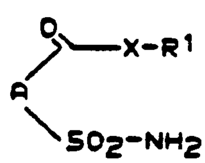

(IV)

with a (thio)carbamate of the formula (V)

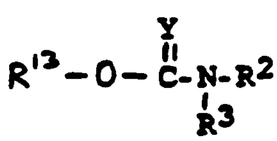

(V),

where $R^{13}$ denotes $(C_1\text{-}C_6)$alkyl, $(C_1\text{-}C_4)$haloalkyl or phenyl which can be monosubstituted or polysubstituted by halogen, $(C_1\text{-}C_4)$alkyl or $NO_2$,

(c) a (thio)carbamate of the formula (VI) with a compound of the formula (III)

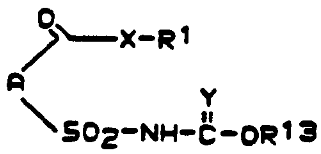

(VI),

where $R^{13}$ has the abovementioned meaning, or
(d) a carboxylic acid of the formula (VII)

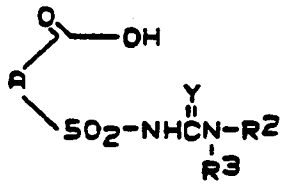

(VII)

with an alkylating reagent of the formula (VIII)

$R^1$-X (VIII)

where X represents a nucleofugic leaving group, and, if appropriate, converting the resulting compound to a salt thereof.

**7.** A herbicidal or plant-growth-regulating agent, which contains an effective amount of a compound of the formula I as claimed in one of claims 1 to 5, a stereoisomer or a salt thereof and inert auxiliaries.

**8.** The use of a compound of the formula I as claimed in one of claims 1 to 5, a stereoisomer or a salt thereof as a herbicide or plant growth regulator.

**9.** A method of controlling noxious plants, which comprises applying an effective amount of a compound of the formula I defined as claimed in one of claims 1 to 5 or a stereoisomer or a salt thereof to these noxious plants or the soils which are used in agriculture or industry.

**10.** A method of regulating the growth of crop plants, which comprises applying an effective amount of a compound of the formula I as claimed in one of claims 1 to 5 or a stereoisomer or a salt thereof to these crop plants or the cropped area.

**Claims for the following Contracting State : ES**

**1.** A process for the preparation of a compound of the formula I

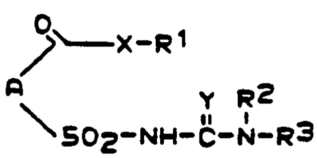

(I)

wherein

A denotes a radical of the formula $>C(R^4)2$ or $>C=C(R^5)_2$,

$R^1$ denotes H, $(C_1-C_8)$alkyl, $(C_2-C_8)$alkenyl, $(C_2-C_8)$alkynyl, it being possible for these aliphatic radicals to be monosubstituted or polysubstituted by halogen, or to be monosubstituted or disubstituted by $(C_1-C_6)$alkoxy, $(C_2-C_6)$alkenyloxy, $(C_2-C_6)$alkynyloxy, $(C_1-C_6)$ alkylthio, $(C_1-C_6)$alkylsulfinyl, $(C_1-C_6)$alkylsulfonyl, $(C_1-C_6)$alkoxycarbonyl or by phenyl; $(C_3-C_8)$cycloalkyl which can be monosubstituted or polysubstituted by halogen, or

monosubstituted or disubstituted by $(C_1\text{-}C_4)$alkoxy or $(C_1\text{-}C_4)$alkylthio; $(C_5\text{-}C_8)$-cycloalkenyl, cyclopropylmethyl, epoxypropyl, furfuryl, tetrahydrofurfuryl, or phenoxy$(C_1\text{-}C_6)$alkyl or phenyl, both of which can be substituted in the phenyl ring by halogen, $(C_1\text{-}C_4)$alkyl, $(C_1\text{-}C_4)$alkoxy or $NO_2$,

$R^2$ denotes H, $(C_1\text{-}C_8)$alkyl, $(C_2\text{-}C_8)$alkenyl, $(C_2\text{-}C_8)$alkynyl or $(C_1\text{-}C_4)$alkoxy,

$R^3$ denotes a radical of the formula

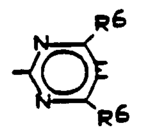 , 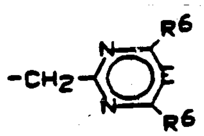 , 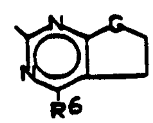 ,

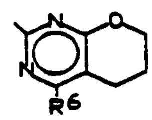 , 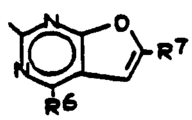 , 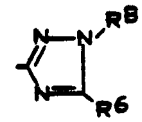 ,

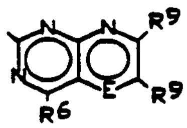 or 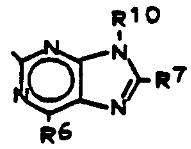

the $R^4$ radicals independently of one another denote H, $(C_1\text{-}C_6)$alkyl, $(C_2\text{-}C_6)$alkenyl, $(C_2\text{-}C_6)$-alkynyl, $(C_3\text{-}C_8)$cycloalkyl, it being possible for the abovementioned radicals which contain C to be monosubstituted or polysubstituted by halogen or monosubstituted or disubstituted by $(C_1\text{-}C_6)$alkoxy, $(C_2\text{-}C_6)$alkenyloxy, $(C_2\text{-}C_6)$alkynyloxy, $(C_1\text{-}C_6)$alkylthio, $(C_1\text{-}C_6)$alkylsulfinyl, $(C_1\text{-}C_6)$alkylsulfonyl or $(C_1\text{-}C_6)$alkoxycarbonyl, or by phenyl which can be monosubstituted or polysubstituted by halogen, $(C_1\text{-}C_4)$alkyl, $(C_1\text{-}C_4)$alkoxy, $(C_1\text{-}C_4)$alkylthio, $(C_1\text{-}C_4)$alkoxycarbonyl or $NO_2$, it being furthermore possible for one of the two radicals $R^4$ to denote phenyl which can be monosubstituted or polysubstituted by halogen, $(C_1\text{-}C_4)$alkyl, $(C_1\text{-}C_4)$alkoxy, $NO_2$ or $CF_3$,

the $R^5$ radicals independently of one another denote H, or $(C_1\text{-}C_6)$alkyl, which can be monosubstituted or polysubstituted by halogen or monosubstituted or disubstituted by $(C_1\text{-}C_6)$-alkoxy, $(C_1\text{-}C_6)$alkylthio, $(C_1\text{-}C_6)$alkylsulfinyl or $(C_1\text{-}C_6)$alkoxycarbonyl, or it being possible for one of the radicals $R^5$ to denote phenyl which can be monosubstituted or polysubstituted by halogen, $(C_1\text{-}C_4)$alkyl, $(C_1\text{-}C_4)$alkoxy, $NO_2$, $CF_3$ or $(C_1\text{-}C_6)$alkoxycarbonyl, or for the two radicals $R^5$ together to denote an alkylene radical $-(CH_2)_n-$,

the $R^6$ radicals independently of one another denote H, halogen, or $(C_1\text{-}C_6)$alkyl, $(C_1\text{-}C_6)$alkoxy or $(C_1\text{-}C_6)$alkylthio which can be monosubstituted or polysubstituted by halogen or monosubstituted or disubstituted by $(C_1\text{-}C_4)$alkoxy or $(C_1\text{-}C_4)$alkylthio; a radical $N(R^{11})_2$, $(C_3\text{-}C_6)$cycloalkyl, $-OCHR^7COOR^{11}$, $(C_3\text{-}C_5)$alkenyl, $(C_2\text{-}C_4)$alkynyl, $(C_3\text{-}C_5)$alkenyloxy or $(C_3\text{-}C_5)$alkynyloxy,

$R^7$ denotes H or $(C_1\text{-}C_4)$alkyl,

$R^8$ denotes $(C_1\text{-}C_4)$alkyl, $-CHF_2$ or $-CH_2CF_3$,

the $R^9$ radicals independently of one another denote H, $(C_1\text{-}C_4)$alkyl, $(C_1\text{-}C_4)$alkoxy or halogen,

$R^{10}$ denotes H, $(C_1\text{-}C_4)$alkyl, $-CHF_2$ or $-CH_2CF_3$,

the $R^{11}$ radicals independently of one another denote H, $(C_1\text{-}C_4)$alkyl, $(C_2\text{-}C_4)$alkenyl or $(C_3\text{-}C_4)$-alkynyl,

E denotes CH or N,

G denotes $CH_2$ or O,

X denotes O, S or $NR^{12}$,

Y denotes O or S,

$R^{12}$ denotes H, $(C_1\text{-}C_6)$alkyl, $(C_2\text{-}C_6)$alkenyl, $(C_2\text{-}C_6)$alkynyl, $(C_1\text{-}C_6)$alkoxy, or a radical of the formula

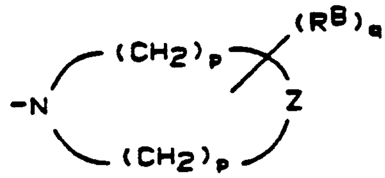

n denotes an integer from 3 to 6,

p denotes an integer from 1 to 3,

q denotes an integer from 0 to 3 and

Z denotes O, S, $CH_2$, NH or N($C_1$-$C_4$-alkyl), or the salts thereof which can be employed in agriculture, which comprises reacting

(a) a compound of the formula (II)

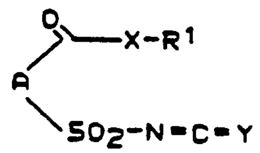

(II)

where A, X, Y, $R^1$ have the meaning indicated in claim 1, with the exception that $R^1$ is not H and X is not NH, with a compound of the formula (III)

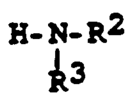

(III)

(b) a compound of the formula (IV)

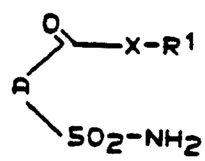

(IV)

with a (thio)carbamate of the formula (V)

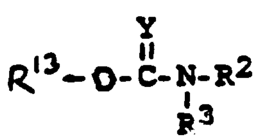

(V),

where $R^{13}$ denotes ($C_1$-$C_6$)alkyl, ($C_1$-$C_4$)haloalkyl or phenyl which can be monosubstituted or polysubstituted by halogen, ($C_1$-$C_4$)alkyl or $NO_2$,

(c) a (thio)carbamate of the formula (VI) with a compound of the formula (III)

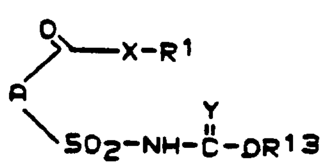

(VI),

where $R^{13}$ has the abovementioned meaning, or
(d) a carboxylic acid of the formula (VII)

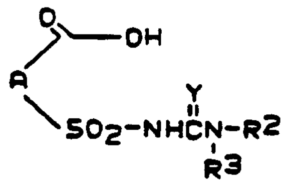

(VII)

with an alkylating reagent of the formula (VIII)

$R^1$-X (VIII)

where X represents a nucleofugic leaving group, and, if appropriate, converting the resulting compound to a salt thereof.

**2.** The process as claimed in claim 1, wherein, in formula (I),

| | |
|---|---|
| A | denotes a radical $>C(R^4)_2$ or $>C = C(R^5)_2$, |
| $R^1$ | denotes $(C_1-C_4)$alkyl which can be monosubstituted or polysubstituted by halogen or monosubstituted to disubstituted by $(C_1-C_4)$alkoxy, |
| $R^2$ | denotes H, $(C_1-C_4)$alkyl or allyl, |
| $R^3$ | denotes a radical of the formula 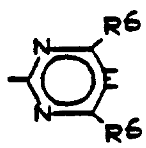 , |
| the $R^4$ | radicals independently of one another denote H, or $(C_1-C_6)$alkyl which can optionally be monosubstituted or polysubstituted by halogen or monosubstituted to disubstituted by $(C_1-C_4)$alkoxy or $(C_1-C_4)$alkylthio, or a radical $R^4$ denotes $(C_2-C_6)$alkenyl or phenyl which can be monosubstituted or polysubstituted by halogen, $(C_1-C_4)$alkyl, $(C_1-C_4)$alkoxy, $NO_2$ or $CF_3$, and the other radical $R^4$ denotes hydrogen, |
| the $R^5$ | radicals independently of one another denote H, or $(C_1-C_6)$alkyl, which can optionally be monosubstituted or polysubstituted by halogen, |
| the $R^6$ | radicals independently of one another denote halogen, $(C_1-C_4)$alkyl or $(C_1-C_4)$alkoxy, both of which can be halogenated, |
| E | denotes CH or N, |
| X | denotes O, S or $NR^{12}$, |
| Y | denotes O or S and |
| $R^{12}$ | denotes H, $(C_1-C_4)$alkyl or $(C_2-C_4)$alkenyl. |

**3.** The process as claimed in claim 1 or 2, wherein one radical $R^4$ is $(C_1-C_4)$-alkyl or is phenyl which is monosubstituted to trisubstituted by fluorine, chlorine, $(C_1-C_4)$-alkoxy, $(C_1-C_4)$-alkyl or nitro, and the other radical $R^4$ is hydrogen.

**4.** The process as claimed in one of claims 1 to 3, wherein R' denotes $(C_1-C_4)$-alkyl and $R^2$ denotes hydrogen.

**5.** The process as claimed in one of claims 1 to 4, wherein $R^6$ denotes $CH_3$, $OCH_3$, $OCH_2H_5$, Cl or $OCF_2H$.

**6.** The use of a compound of the formula I as defined in one of claims 1 to 5, a stereoisomer or a salt thereof as a herbicide or plant growth regulator.

7. A method of controlling noxious plants, which comprises applying an effective amount of a compound of the formula I as defined in one of claims 1 to 5 or a stereoisomer or a salt thereof to these noxious plants or the soils which are used in agriculture or industry.

8. A method of regulating the growth of crop plants, which comprises applying an effective amount of a compound of the formula I as defined in one of claims 1 to 5 or a stereoisomer or a salt thereof to these crop plants or the cropped area.

**Revendications**

**Revendications pour les Etats contractants suivants : AT, BE, CH, DE, FR, GB, IT, LI, NL, SE**

1. Composés de formule (I)

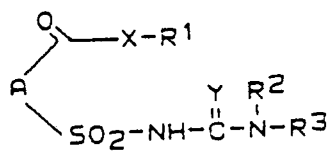

(I) ,

dans laquelle

A est un résidu de formule $>C(R^4)_2$ ou $>C=C(R^5)_2$,

$R^1$ est H ou un radical alkyle en $C_1$-$C_8$, alcényle en $C_2$-$C_8$, alcynyle en $C_2$-$C_8$, ces résidus aliphatiques pouvant être une ou plusieurs fois substitués par des halogènes ou une ou deux fois par des substituants alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, (alcoxy en $C_1$-$C_6$)carbonyle, ou par des substituants phényle ; un radical cycloalkyle en $C_3$-$C_8$, pouvant être une ou plusieurs fois substitué par des halogènes ou une ou deux fois par des substituants alcoxy en $C_1$-$C_4$ ou alkylthio en $C_1$-$C_4$ ; cycloalcényle en $C_5$-$C_8$, cyclopropylméthyle, époxypropyle, furfuryle, tétrahydrofurfuryle, phénoxy-(alkyle en $C_1$-$C_6$) ou phényle, qui tous les deux peuvent être substitués dans le noyau phényle par des substituants halogéno, alkyle en $C_1$-$C_4$, alcoxy en $C_1$-$C_4$, ou $NO_2$,

$R^2$ est H ou un radical alkyle en $C_1$-$C_8$, alcényle en $C_2$-$C_8$, alcynyle en $C_2$-$C_8$ ou alcoxy en $C_1$-$C_4$,

$R^3$ est un résidu de formule :

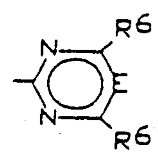 , 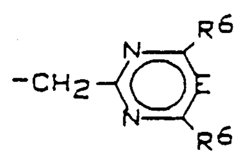 , 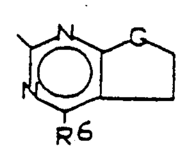 ,

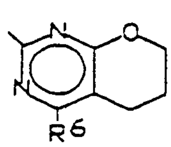 , 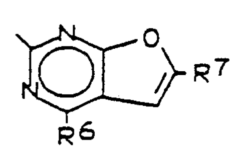 , 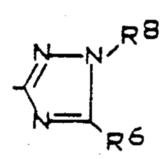 ,

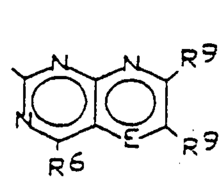 ou 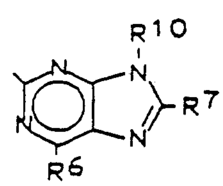

les radicaux $R^4$, indépendamment l'un de l'autre, représentent chacun H ou un radical alkyle en $C_1$-$C_6$, alcényle en $C_2$-$C_6$, alcynyle en $C_2$-$C_6$, cycloalkyle en $C_3$-$C_8$, les radicaux carbonés mentionnés ci-dessus pouvant être substitués une ou plusieurs fois par des halogènes ou une ou deux fois par des substituants alcoxy en $C_1$-$C_6$,alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$ ou (alcoxy en $C_1$-$C_6$)carbonyle, ou encore par un radical

phényle pouvant être lui-même substitué une ou plusieurs fois par des halogènes ou des substituants alkyle en $C_1$-$C_4$, alcoxy en $C_1$-$C_4$, alkylthio en $C_1$-$C_4$, (alcoxy en $C_1$-$C_4$)carbonyle ou $NO_2$, et de plus l'un des deux radicaux $R^4$ est un radical phényle pouvant être une ou plusieurs fois substitué par des halogènes ou des radicaux alkyle en $C_1$-$C_4$, alcoxy en $C_1$-$C_4$, $NO_2$ ou $CF_3$,

les radicaux $R^5$, indépendamment l'un de l'autre, représentent chacun H ou un radical alkyle en $C_1$-$C_6$, qui peut être une ou plusieurs fois substitué par des halogènes ou une ou deux fois par des substituants alcoxy en $C_1$-$C_6$, alkylthio en $C_1$-$C_6$, alkylsulfinyle en $C_1$-$C_6$(alcoxy en $C_1$-$C_6$)carbonyle, ou encore l'un des radicaux $R^5$ est un radical phényle pouvant être une ou plusieurs fois substitué par des radicaux halogéno, alkyle en $C_1$-$C_4$,alcoxy en $C_1$-$C_4$, $NO_2$, $CF_3$ (alcoxy en $C_1$-$C_6$)carbonyle ou encore les deux radicaux $R^5$ forment ensemble un radical alkylène $-(CH_2)_n-$,

les radicaux $R^6$, indépendamment l'un de l'autre, représentent chacun H ou un halogène, un radical alkyle en $C_1$-$C_6$, alcoxy en $C_1$-$C_6$, ou alkylthio en $C_1$-$C_6$, pouvant être substitué une ou plusieurs fois par des halogènes ou une ou deux fois par des substituants alcoxy en $C_1$-$C_4$ ou alkylthio en $C_1$-$C_4$ ; un radical $N(R^{11})_2$, cycloalkyle en $C_3$-$C_6$, $-CHR^7COOR^{11}$, alcényle en $C_3$-$C_5$, alcynyle en $C_2$-$C_4$, alcényloxy en $C_3$-$C_5$ ou alcynyloxy en $C_3$-$C_5$,

$R^7$ est H ou un radical alkyle en $C_1$-$C_4$,

$R^8$ est un radical alkyle en $C_1$-$C_4$, $-CHF_2$ ou $-CH_2CF_3$,

$R^9$, indépendamment l'un de l'autre, représente H ou un radical alkyle en $C_1$-$C_4$, alcoxy en $C_1$-$C_4$ ou halogéno,

$R^{10}$ est H ou un radical alkyle en $C_1$-$C_4$, $-CHF_2$ ou $-CH_2CF_3$,

les radicaux $R^{11}$, indépendamment l'un de l'autre, représentent chacun H ou un radical alkyle en $C_1$-$C_4$, alcényle en $C_2$-$C_4$ ou alcynyle en $C_3$-$C_4$,

E est CH ou N,

G est $CH_2$ ou O,

X est O, S ou $NR^{12}$,

Y est O ou S,

$R^{12}$ est H ou un radical alkyle en $C_1$-$C_6$, alcényle en $C_2$-$C_6$, alcynyle en $C_2$-$C_6$, alcoxy en $C_1$-$C_6$, ou un résidu de formule

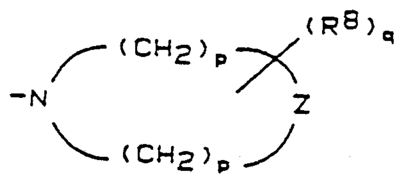

n est un nombre entier de 3 à 6,

p est un nombre entier de 1 à 3,

q est un nombre entier de 0 à 3,

z est O, S, $CH_2$, NH ou N(alkyle en $C_1$-$C_4$), ou leurs sels pouvant être utilisés en agriculture.

**2.** Composés de formule (I) selon la revendication 1, caractérisés en ce que A est un résidu de formule $>C(R^4)_2$ ou $>C=C(R^5)_2$,

$R^1$ est un radical alkyle en $C_1$-$C_4$, pouvant être une ou plusieurs fois substitué par des halogènes ou une ou deux fois par des substituants alcoxy en $C_1$-$C_4$,

$R^2$ est H ou un radical alkyle en $C_1$-$C_4$, ou allyle,

$R^3$ est un radical de formule

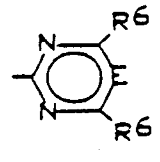 ,

les radicaux $R^4$, indépendamment l'un de l'autre, sont chacun H ou un radical alkyle en $C_1$-$C_6$, pouvant être éventuellement substitué une ou plusieurs fois par des halogènes ou une ou deux fois par

des substituants alcoxy en $C_1$-$C_4$ ou alkylthio en $C_1$-$C_4$, ou encore un radical $R^4$ est un radical alcényle en $C_2$-$C_6$ ou phényle, qui peut être substitué une ou plusieurs fois par des halogènes ou des radicaux alkyle en $C_1$-$C_4$, alcoxy en $C_1$-$C_4$, $NO_2$ ou $CF_3$, en particulier, un radical $R^4$ est un radical alkyle en $C_1$-$C_4$ ou phényle, et l'autre radical $R^4$ est un hydrogéne,

les radicaux $R^5$, indépendamment l'un de l'autre, sont chacun H ou un radical alkyle en $C_1$-$C_6$, qui peut être éventuellement une ou plusieurs fois substitué par des halogènes,

les radicaux $R^6$, indépendamment l'un de l'autre, sont chacun un halogène ou un radical alkyle en $C_1$-$C_4$ oualcoxy en $C_1$-$C_4$, qui tous les deux peuvent être halogénés,

E est CH ou N,

X est O, S ou $NR^{12}$,

Y est O ou S, et

$R^{12}$ est H ou un radical alkyle en $C_1$-$C_4$ ou alcényle en $C_2$-$C_4$.

**3.** Composés selon les revendications 1 ou 2, caractérisés en ce qu'un radical $R^4$ est un radical alkyle en $C_1$-$C_4$ ou le radical phényle, substitué une à trois fois par des substituants fluoro, chloro, alcoxy en $C_1$-$C_4$, alkyle en $C_1$-$C_4$ ou nitro, et l'autre radical $R^4$ est un hydrogène.

**4.** Composés selon l'une des revendications 1 à 3, caractérisés en ce que $R^1$ est un radical alkyle en $C_1$-$C_4$ et $R^2$ est un hydrogène.

**5.** Composés selon l'une des revendications 1 à 4, caractérisés en ce que $R^6$ est $CH_3$, $OCH_3$, $OC_2H_5$, Cl ou $OCF_2H$.

**6.** Procédé pour préparer les composés de formule (I) selon la revendication (I) ou leurs sels, caractérisé en ce que

a) on fait réagir un composé de formule (II)

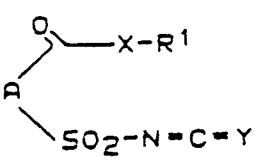
(II) ,

dans laquelle A, X, Y et $R^1$ ont les significations données ci-dessus, sauf que $R^1$ est différent de H et X est différent de NH, avec un composé de formule (III)

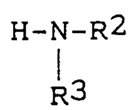
(III)

b) on fait réagir un composé de formule (IV)

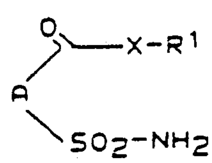
(IV)

avec un (thio)-carbamate de formule (V)

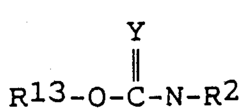
(V)

où $R^{13}$ est un radical alkyle en $C_1$-$C_6$, halogénalkyle en $C_1$-$C_4$ ou phényle, pouvant être une ou plusieurs fois substitué par des halogènes ou des substituants alkyle en $C_1$-$C_4$ ou $NO_2$,

c) on fait réagir un (thio)-carbamate de formule (VI) avec un composé de formule (III)

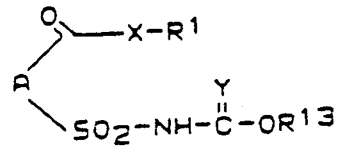

(VI),

dans laquelle $R^{13}$ a les significations données ci-dessus, ou bien

d) on fait réagir un acide carboxylique de formule (VII)

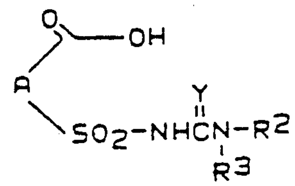

(VII)

avec un réactif d'alkylation de formule (VIII)

$R^1$-X (VIII)

où X est un groupe éliminable nucléofuge, tel par exemple un groupe halogéno, alkyl-$SO_2$-O- ou tosyle, et on convertit éventuellement les composés ainsi obtenus en leurs sels.

**7.** Herbicides ou agents régulateurs de croissance des végétaux, caractérisés en ce qu'ils contiennent une quantité efficace d'un composé de formule (I) selon l'une des revendications 1 à 5, de leurs stéréo-isomères ou de leurs sels, et d'adjuvants inertes.

**8.** Utilisation des composés de formule (I) selon l'une des revendications 1 à 5, de leurs stéréo-isomères ou de leurs sels, comme herbicides ou régulateurs de croissance des végétaux.

**9.** Procédé pour lutter contre les plantes nuisibles, caractérisé en ce qu'on applique sur ces dernières ou sur les sols à utilisation agricole ou industrielle, une quantité efficace d'un composé défini dans l'une des revendications 1 à 5, de formule (I) ou de leurs stéréoisomères ou de leurs sels.

**10.** Procédé de régulation de la croissance de plantes utiles, caractérisé en ce qu'on applique sur ces dernières, ou sur la surface de culture, une quantité efficace d'un composé de formule (I) selon l'une des revendications 1 à 5, ou de leurs stéréo-isomères, ou de leurs sels.

**Revendications pour l'Etat contractant suivant : ES**

**1.** Procédé pour préparer des composés de formule (I)

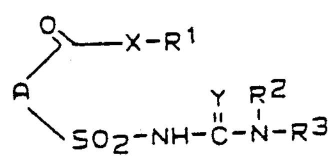

(I) ,

dans laquelle

A est un résidu de formule >C($R^4$)$_2$ ou >C=C($R^5$)$_2$,

$R^1$ est H ou un radical alkyle en $C_1$-$C_8$, alcényle en $C_2$-$C_8$, alcynyle en $C_2$-$C_8$, ses résidus

aliphatiques pouvant être une ou plusieurs fois substitués par des halogènes ou une ou deux fois par des substituants alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, (alcoxy en $C_1$-$C_6$)carbonyle, ou par des substituants phényle ; un radical cycloalkyle en $C_3$-$C_8$, pouvant être une ou plusieurs fois substitué par des halogènes ou une ou deux fois par des substituants alcoxy en $C_1$-$C_4$ ou alkylthio en $C_1$-$C_4$ ; cycloalcényle en $C_5$-$C_8$, cyclopropylméthyle, époxypropyle, furfuryle, tétrahydrofurfuryle, phénoxy-(alkyle en $C_1$-$C_6$) ou phényle, qui tous les deux peuvent être substitués dans le noyau phényle par des substituants halogéno, alkyle en $C_1$-$C_4$, alcoxy en $C_1$-$C_4$, ou $NO_2$,

$R^2$ est H ou un radical alkyle en $C_1$-$C_8$, alcényle en $C_2$-$C_8$, alcynyle en $C_2$-$C_8$ ou alcoxy en $C_1$-$C_4$,

$R^3$ est un résidu de formule :

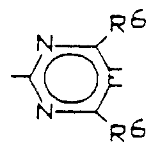 , 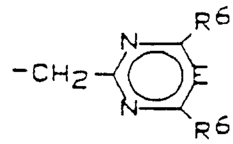 , 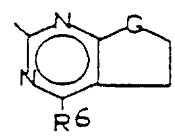 ,

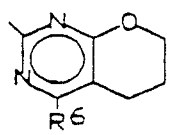 , 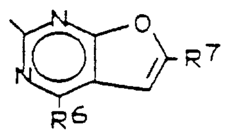 , 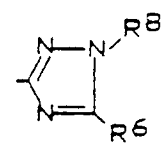 ,

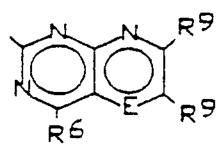 ou 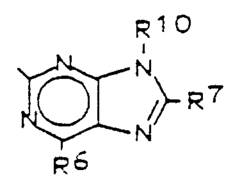

les radicaux $R^4$, independamment l'un de l'autre, représentent chacun H ou un radical alkyle en $C_1$-$C_6$, alcényle en $C_2$-$C_6$, alcynyle en $C_2$-$C_6$, cycloalkyle en $C_3$-$C_8$, les radicaux carbonés mentionnés ci-dessus pouvant être substitués une ou plusieurs fois par des halogènes ou une ou deux fois par des substituants alcoxy en $C_1$-$C_6$,alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$ ou (alcoxy en $C_1$-$C_6$)carbonyle, ou encore par un radical phényle pouvant être lui-même substitué une ou plusieurs fois par des halogènes ou des substituants alkyle en $C_1$-$C_4$, alcoxy en $C_1$-$C_4$, alkylthio en $C_1$-$C_4$, (alcoxy en $C_1$-$C_4$)carbonyle ou $NO_2$, et de plus l'un des deux radicaux $R^4$ est un radical phényle pouvant être une ou plusieurs fois substitué par des halogènes ou des radicaux alkyle en $C_1$-$C_4$, alcoxy en $C_1$-$C_4$, $NO_2$ ou $CF_3$,

les radicaux $R^5$, indépendamment l'un de l'autre, représentent chacun H ou un radical alkyle en $C_1$-$C_6$, qui peut être une ou plusieurs fois substitué par des halogènes ou une ou deux fois par des substituants alcoxy en $C_1$-$C_6$, alkylthio en $C_1$-$C_6$, alkylsulfinyle en $C_1$-$C_6$(alcoxy en $C_1$-$C_6$)carbonyle, ou encore l'un des radicaux $R^5$ est un radical phényle pouvant être une ou plusieurs fois substitué par des radicaux halogéno, alkyle en $C_1$-$C_4$,alcoxy en $C_1$-$C_4$, $NO_2$, $CF_3$ (alcoxy en $C_1$-$C_6$)carbonyle ou encore les deux radicaux $R^5$ forment ensemble un radical alkylène $-(CH_2)_n-$,

les radicaux $R^6$, indépendamment l'un de l'autre, représentent chacun H ou un halogène, un radical alkyle en $C_1$-$C_6$, alcoxy en $C_1$-$C_6$, ou alkylthio en $C_1$-$C_6$, pouvant être substitué une ou plusieurs fois par des halogènes ou une ou deux fois par des substituants alcoxy en $C_1$-$C_4$ ou alkylthio en $C_1$-$C_4$ ; un radical $N(R^{11})_2$, cycloalkyle en $C_3$-$C_6$, $-CHR^7COOR^{11}$, alcényle en $C_3$-$C_5$, alcynyle en $C_2$-$C_4$, alcényloxy en $C_3$-$C_5$ ou alcynyloxy en $C_3$-$C_5$,

$R^7$ est H ou un radical alkyle en $C_1$-$C_4$,

$R^8$ est un radical alkyle en $C_1$-$C_4$, $-CHF_2$ ou $-CH_2CF_3$,

$R^9$, indépendamment l'un de l'autre, représente H ou un radical alkyle en $C_1$-$C_4$, alcoxy en $C_1$-$C_4$ ou halogéno,

$R^{10}$ est H ou un radical alkyle en $C_1$-$C_4$, $-CHF_2$ ou $-CH_2CF_3$,

les radicaux $R^{11}$, indépendamment l'un de l'autre, représentent chacun H ou un radical alkyle en $C_1$-$C_4$, alcényle en $C_2$-$C_4$ ou alcynyle en $C_3$-$C_4$,

E est CH ou N,

G est $CH_2$ ou O,
X est O, S ou $NR^{12}$,
Y est O ou S,
$R^{12}$ est H ou un radical alkyle en $C_1$-$C_6$, alcényle en $C_2$-$C_6$, alcynyle en $C_2$-$C_6$, alcoxy en $C_1$-$C_6$, ou un résidu de formule

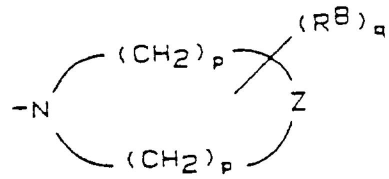

n est un nombre entier de 3 à 6,
p est un nombre entier de 1 à 3,
q est un nombre entier de 0 à 3,
z est O, S, $CH_2$, NH ou N(alkyle en $C_1$-$C_4$), ou leurs sels pouvant être utilisés en agriculture, caractérisés en ce que

a) on fait réagir un composé de formule (II)

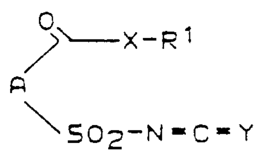

(II) ,

dans laquelle A, X, Y et $R^1$ ont les significations données ci-dessus, sauf que $R^1$ est différent de H et X est différent de NH, avec un composé de formule (III)

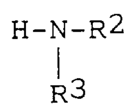

(III)

b) on fait réagir un composé de formule (IV)

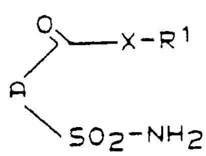

(IV)

avec un (thio)-carbamate de formule (V)

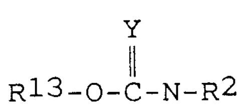

(V)

où $R^{13}$ est un radical alkyle en $C_1$-$C_6$, halogénalkyle en $C_1$-$C_4$ ou phényle, pouvant être une ou plusieurs fois substitué par des halogènes ou des substituants alkyle en $C_1$-$C_4$ ou $NO_2$,

c) on fait réagir un (thio)-carbamate de formule (VI) avec un composé de formule (III)

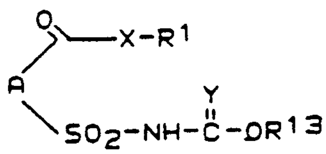

(VI),

dans laquelle $R^{13}$ a les significations données ci-dessus, ou bien
d) on fait réagir un acide carboxylique de formule (VII)

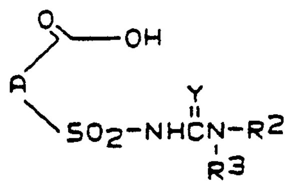

(VII)

avec un réactif d'alkylation de formule (VIII)

$R^1$-X (VIII)

où X est un groupe éliminable nucléofuge, tel par exemple un groupe halogéno, alkyl-$SO_2$-O- ou tosyle, et on convertit éventuellement les composés ainsi obtenus en leurs sels.

**2.** Procédé selon la revendication 1, caractérisé en ce que, dans la formule (I)
A est un résidu de formule $>C(R^4)_2$ ou $>C=C(R^5)_2$,
$R^1$ est un radical alkyle en $C_1$-$C_4$, pouvant être une ou plusieurs fois substitué par des halogènes ou une ou deux fois par des substituants alcoxy en $C_1$-$C_4$,
$R^2$ est H ou un radical alkyle en $C_1$-$C_4$, ou allyle,
$R^3$ est un radical de formule

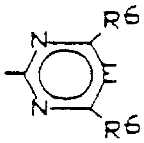

,

les radicaux $R^4$, indépendamment l'un de l'autre, sont chacun H ou un radical alkyle en $C_1$-$C_6$, pouvant être éventuellement substitué une ou plusieurs fois par des halogènes ou une ou deux fois par des substituants alcoxy en $C_1$-$C_4$ ou alkylthio en $C_1$-$C_4$, ou encore un radical $R^4$ est un radical alcényle en $C_2$-$C_6$ ou phényle, qui peut être substitué une ou plusieurs fois par des halogènes ou des radicaux alkyle en $C_1$-$C_4$, alcoxy en $C_1$-$C_4$, $NO_2$ ou $CF_3$, en particulier, un radical $R^4$ est un radical alkyle en $C_1$-$C_4$ ou phényle, et l'autre radical $R^4$ est un hydrogéne,
les radicaux $R^5$, indépendamment l'un de l'autre, sont chacun H ou un radical alkyle en $C_1$-$C_6$, qui peut être éventuellement une ou plusieurs fois substitué par des halogènes,
les radicaux $R^6$, indépendamment l'un de l'autre, sont chacun un halogéne ou un radical alkyle en $C_1$-$C_4$ oualcoxy en $C_1$-$C_4$, qui tous les deux peuvent être halogénés,
E est CH ou N,
X est O, S ou $NR^{12}$,
Y est O ou S, et
$R^{12}$ est H ou un radical alkyle en $C_1$-$C_4$ ou alcényle en $C_2$-$C_4$.

**3.** Procédé selon les revendications 1 ou 2, caractérisé en ce qu'un radical $R^4$ est un radical alkyle en $C_1$-$C_4$ ou le radical phényle, substitué à trois fois par des substituants fluoro, chloro, alcoxy en $C_1$-$C_4$,

alkyle en $C_1$-$C_4$ ou nitro, et l'autre radical $R^4$ est un hydrogène.

**4.** Procédé selon l'une des revendications 1 à 3, caractérisé en ce que $R^1$ est un radical alkyle en $C_1$-$C_4$ et $R^2$ est un hydrogène.

**5.** Procédé selon l'une des revendications 1 à 4, caractérisé en ce que $R^6$ est $CH_3$, $OCH_3$, $OC_2H_5$, Cl ou $OCF_2H$.

**6.** Utilisation des composés de formule (I) selon l'une des revendications 1 à 5, de leurs stéréo-isomères ou de leurs sels, comme herbicides ou régulateurs de croissance des végétaux.

**7.** Procédé pour lutter contre les plantes nuisibles, caractérisé en ce qu'on applique sur ces dernières ou sur les sols à utilisation agricole ou industrielle, une quantité efficace d'un composé défini dans l'une des revendications 1 à 5, de formule (I) ou de leurs stéréoisomères ou de leurs sels.

**8.** Procédé de régulation de la croissance de plantes utiles, caractérisé en ce qu'on applique sur ces dernières, ou sur la surface de culture, une quantité efficace d'un composé de formule (I) selon l'une des revendications 1 à 5, ou de leurs stéréo-isomères, ou de leurs sels.